(19) Europäisches Patentamt
European Patent Office
Office européen des brevets

(11) **EP 4 397 304 A1**

(12) **EUROPEAN PATENT APPLICATION**
published in accordance with Art. 153(4) EPC

(43) Date of publication:
**10.07.2024 Bulletin 2024/28**

(21) Application number: **22865019.8**

(22) Date of filing: **30.08.2022**

(51) International Patent Classification (IPC):
***A61K 31/122*** *(2006.01)* ***A61P 3/04*** *(2006.01)*
***A61P 1/16*** *(2006.01)*

(52) Cooperative Patent Classification (CPC):
**A23L 33/10; A61K 31/122; A61P 1/16; A61P 3/04**

(86) International application number:
**PCT/KR2022/012956**

(87) International publication number:
**WO 2023/033517 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.08.2021 KR 20210114751**

(71) Applicant: **Shin Poong Pharmaceutical Co., Ltd.**
**Ansan-si, Gyeonggi-do 15610 (KR)**

(72) Inventors:
- **JU, Chung**
  **Seoul 06767 (KR)**
- **LEE, Dong Won**
  **Gunpo-si Gyeonggi-do 15821 (KR)**
- **LEE, Seung Kyu**
  **Suwon-si Gyeonggi-do 16689 (KR)**
- **CHUNG, Sung**
  **Seoul 08078 (KR)**

(74) Representative: **Mewburn Ellis LLP**
**Aurora Building**
**Counterslip**
**Bristol BS1 6BX (GB)**

(54) **COMPOSITION FOR TREATING OR PREVENTING OBESITY OR OBESITY-ASSOCIATED LIVER DISEASE, COMPRISING VERBENONE DERIVATIVES**

(57) The present invention relates to a pharmaceutical composition or a health functional food for treating or preventing obesity or obesity-related liver disease, containing a verbenone derivative and a pharmaceutically acceptable salt thereof as an active ingredient. Specifically, the verbenone derivative of the present invention is capable of treating and preventing obesity or obesity-related liver disease by inhibiting TGF-β/Smad3 signaling in high-fat diet-induced mice.

FIG. 1

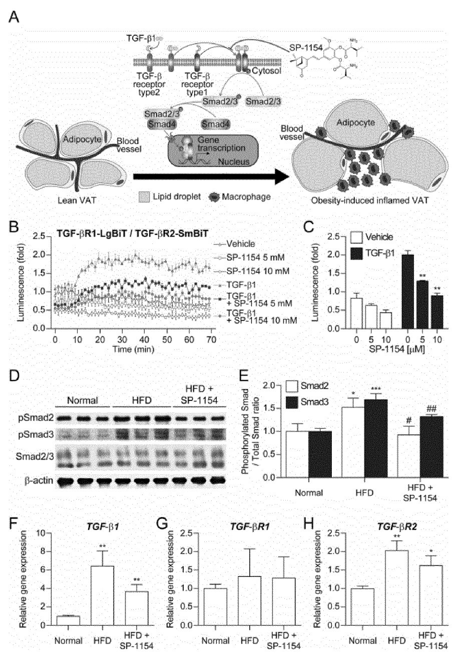




EP 4 397 304 A1

## Description

### Technical Field

The present invention relates to a pharmaceutical composition for treating or preventing obesity or obesity-related liver disease containing a verbenone derivative, and more specifically, to a pharmaceutical composition for treating or preventing obesity or obesity-related liver disease containing a verbenone derivative, which is capable of treating or preventing obesity or obesity-related liver disease by inhibiting TGF-β/Smad3 signaling in high-fat diet-induced mice.

### Background Art

Obesity-related non-alcoholic fatty liver disease (NAFLD), a continuum of chronic liver disease from non-alcoholic fatty liver to non-alcoholic steatohepatitis (NASH), is the leading cause of liver-related morbidity and mortality. Furthermore, the prevalence of NALFD is approximately estimated at 25% of the global general population and its prevalence is continuously rising, thereby imposing a significant public health burden [2, 3].

Accumulating evidence suggests that the liver closely interacts with visceral adipose tissue (VAT) and the obesity-induced inflamed VAT plays key roles in the development of NAFLD [1, 4-6]. Obesity, especially visceral obesity, promotes the transformation of metabolically healthy VAT into dysregulated and inflamed VAT [5]. Dysregulated VAT, as an endocrine and paracrine organ, secretes pro-inflammatory cytokines such as interleukin-6 (IL-6) and tumor necrosis factor-alpha (TNF-α), thereby increasing the infiltration of inflammatory cells such as macrophages, which lead to the further exacerbation of the inflammation in VAT [4-6]. The subsequent inflamed VAT enhances the secretion of pro-inflammatory cytokines, thereby promoting systemic inflammation and insulin resistance, which further lead to accelerated NAFLD progression [1, 4-6]. Thus, an inflammatory reaction occurring in VAT promotes the NAFLD progression, and would be an appropriate therapeutic target for obesity-related NAFLD [6, 7].

Several previous studies have reported that transforming growth factor (TGF)-β/Smad3 signaling plays crucial roles in the pathological process of VAT inflammation [8-10]. It is known that the interaction between TGF-β1 and a complex of TGF-β type 1 and type 2 receptors (TGF-βR1 and TGF-βR2) activates the phosphorylation of Smad2/3, thereby upregulating VAT inflammation (FIG. 1A) [10]. Furthermore, previous reported results of animal studies found that genetic ablation of Smad3 and treatment with a TGF-β functional blocking antibody reduce obesity-driven VAT inflammation [11, 12]. Thus, the TGF-β/Smad3 signaling pathway could be a therapeutic target for inflamed VAT.

Accordingly, the present inventors made efforts to utilize the TGF-β/Smad3 signaling pathway as a therapeutic target for NAFLD caused by the above-mentioned inflamed VAT, and as a result, a verbenone derivative compound can treat and prevent obesity-induced visceral adipose tissue (VAT) inflammation and subsequent obesity or non-alcoholic fatty liver disease (NAFLD) by inhibiting the TGF-β/Smad3 signaling pathway in high-fat diet-induced (HFD) mice, thereby completing the present invention.

[Prior Art Documents]

[Non-Patent Documents]

[1] Byrne CD, Targher G. NAFLD: a multisystem disease. Journal of hepatology. 2015;62:S47-S64.

[2] Younossi ZM, Blissett D, Blissett R, Henry L, Stepanova M, Younossi Y, et al. The economic and clinical burden of nonalcoholic fatty liver disease in the United States and Europe. Hepatology. 2016;64:1577-86.

[3] Younossi ZM, Koenig AB, Abdelatif D, Fazel Y, Henry L, Wymer M. Global epidemiology of nonalcoholic fatty liver disease-meta-analytic assessment of prevalence, incidence, and outcomes. Hepatology. 2016;64:73-84.

[4] Khan RS, Bril F, Cusi K, Newsome PN. Modulation of insulin resistance in nonalcoholic fatty liver disease. Hepatology. 2019;70:711-24.

[5] Azzu V, Vacca M, Virtue S, Allison M, Vidal-Puig A. Adipose tissue-liver cross talk in the control of whole-body metabolism: implications in non-alcoholic fatty liver disease. Gastroenterology. 2020.

[6] Friedman SL, Neuschwander-Tetri BA, Rinella M, Sanyal AJ. Mechanisms of NAFLD development and therapeutic strategies. Nature medicine. 2018;24:908-22.

[7] Wang Y, Tang B, Long L, Luo P, Xiang W, Li X, et al. Improvement of obesity-associated disorders by a small-molecule drug targeting mitochondria of adipose tissue macrophages. Nature communications. 2021;12:1-16.

[8] Lee M-J. Transforming growth factor beta superfamily regulation of adipose tissue biology in obesity. Biochimica et Biophysica Acta (BBA)-Molecular Basis of Disease. 2018;1864:1160-71.

[9] Sousa-Pinto B, Goncalves L, Rodrigues AR, Tada I, Almeida H, Neves D, et al. Characterization of TGF-β expression and signaling profile in the adipose tissue of rats fed with high-fat and energy-restricted diets. The Journal of nutritional biochemistry. 2016;38:107-15.

[10] Tan C, Chong H, Tan E, Tan N. Getting 'Smad' about obesity and diabetes. Nutrition & diabetes. 2012;2:e29-e.

[11] Tan CK, Leuenberger N, Tan MJ, Yan YW, Chen Y, Kambadur R, et al. Smad3 deficiency in mice protects against insulin resistance and obesity induced by a high-fat diet. Diabetes. 2011;60:464-76.

[12] Yadav H, Quijano C, Kamaraju AK, Gavrilova O, Malek R, Chen W, et al. Protection from obesity and diabetes by blockade of TGF-β/Smad3 signaling. Cell metabolism. 2011;14:67-79.

[13] Ju C, Song S, Hwang S, Kim C, Kim M, Gu J, et al. Discovery of novel (1S)-(-)-verbenone derivatives with anti-oxidant and anti-ischemic effects. Bioorganic & medicinal chemistry letters. 2013;23:5421-5.

[14] Nguyen HT, Reyes-Alcaraz A, Yong HJ, Nguyen LP, Park H-K, Inoue A, et al. CXCR7: a β-arrestin-biased receptor that potentiates cell migration and recruits β-arrestin2 exclusively through Gβγ subunits and GRK2. Cell & bioscience. 2020;10:1-19.

[15] Bachmanov AA, Reed DR, Beauchamp GK, Tordoff MG. Food intake, water intake, and drinking spout side preference of 28 mouse strains. Behavior genetics. 2002;32:435-43.

[16] Pahk K, Kim EJ, Joung C, Seo HS, Kim S. Exercise training reduces inflammatory metabolic activity of visceral fat assessed by 18F-FDG PET/CT in obese women. Clinical Endocrinology. 2020.

[17] Pahk K, Kim EJ, Joung C, Seo HS, Kim S. Association of glucose uptake of visceral fat and acute myocardial infarction: a pilot 18 F-FDG PET/CT study. Cardiovascular Diabetology. 2020;19:1-11.

[18] Jimenez-Gomez Y, Mattison JA, Pearson KJ, Martin-Montalvo A, Palacios HH, Sossong AM, et al. Resveratrol improves adipose insulin signaling and reduces the inflammatory response in adipose tissue of rhesus monkeys on high-fat, high-sugar diet. Cell metabolism. 2013;18:533-45.

[19] Lee ES, Kwon M-H, Kim HM, Woo HB, Ahn CM, Chung CH. Curcumin analog CUR5-8 ameliorates nonalcoholic fatty liver disease in mice with high-fat diet-induced obesity. Metabolism. 2020;103:154015.

[20] Livak KJ, Schmittgen TD. Analysis of relative gene expression data using real-time quantitative PCR and the 2- ΔΔCT method, methods. 2001;25:402-8.

[21] Elsegood CL, Chang M, Jessup W, Scholz GM, Hamilton JA. Glucose Metabolism Is Required for Oxidized LDL-Induced Macrophage Survival: Role of PI3K and Bcl-2 Family Proteins. Arteriosclerosis, thrombosis, and vascular biology. 2009;29:1283-9.

[22] Moraes-Vieira PM, Saghatelian A, Kahn BB. GLUT4 expression in adipocytes regulates de novo lipogenesis and levels of a novel class of lipids with antidiabetic and anti-inflammatory effects. Diabetes. 2016;65:1808-15.

[23] Viglino D, Martin M, Almeras N, Després J-P, Coxson HO, Pépin J-L, et al. Low Liver Density Is Linked to Cardiovascular Comorbidity in COPD: An ECLIPSE Cohort Analysis. International Journal of Chronic Obstructive Pulmonary Disease. 2019;14:3053.

[24] Um MY, Moon MK, Ahn J, Ha TY. Coumarin attenuates hepatic steatosis by down-regulating lipogenic gene expression in mice fed a high-fat diet. British journal of nutrition. 2013;109:1590-7.

[25] Feng D, Zou J, Su D, Mai H, Zhang S, Li P, et al. Curcumin prevents high-fat diet-induced hepatic steatosis in ApoE-/- mice by improving intestinal barrier function and reducing endotoxin and liver TLR4/NF-κB inflammation. Nutrition & metabolism. 2019;16:1-11.

[26] Cildir G, Akincilar SC, Tergaonkar V. Chronic adipose tissue inflammation: all immune cells on the stage. Trends in molecular medicine. 2013;19:487-500.

[27] Xie L, Ortega MT, Mora S, Chapes SK. Interactive changes between macrophages and adipocytes. Clinical and Vaccine Immunology. 2010;17:651-9.

[28] Lumeng CN, Deyoung SM, Saltiel AR. Macrophages block insulin action in adipocytes by altering expression of signaling and glucose transport proteins. American Journal of Physiology-Endocrinology and Metabolism. 2007;292:E166-E74.

[29] Chalasani N, Younossi Z, Lavine JE, Charlton M, Cusi K, Rinella M, et al. The diagnosis and management of nonalcoholic fatty liver disease: practice guidance from the American Association for the Study of Liver Diseases. Hepatology. 2018;67:328-57.

[30] Adams LA, Lymp JF, Sauver JS, Sanderson SO, Lindor KD, Feldstein A, et al. The natural history of nonalcoholic fatty liver disease: a population-based cohort study. Gastroenterology. 2005;129:113-21.

[31] Musolino V, Gliozzi M, Scarano F, Bosco F, Scicchitano M, Nucera S, et al. Bergamot polyphenols improve dyslipidemia and pathophysiological features in a mouse model of non-alcoholic fatty liver disease. Scientific reports. 2020;10:1-14.

[32] Nam J-S, Terabe M, Mamura M, Kang M-J, Chae H, Stuelten C, et al. An anti-transforming growth factor β antibody suppresses metastasis via cooperative effects on multiple cell compartments. Cancer research. 2008;68:3835-43.

[33] Takahashi K, Akatsu Y, Podyma-Inoue KA, Matsumoto T, Takahashi H, Yoshimatsu Y, et al. Targeting all

transforming growth factor-β isoforms with an Fc chimeric receptor impairs tumor growth and angiogenesis of oral squamous cell cancer. Journal of Biological Chemistry. 2020;295:12559-72.
[34] Chames P, Van Regenmortel M, Weiss E, Baty D. Therapeutic antibodies: successes, limitations and hopes for the future. British journal of pharmacology. 2009;157:220-33.
[35] Bell MR, Engleka MJ, Malik A, Strickler JE. To fuse or not to fuse: what is your purpose? Protein Science. 2013;22:1466-77.
[36] Santiago B, Gutierrez-Cañas I, Dotor J, Palao G, Lasarte JJ, Ruiz J, et al. Topical application of a peptide inhibitor of transforming growth factor-β1 ameliorates bleomycin-induced skin fibrosis. Journal of Investigative Dermatology. 2005;125:450-5.
[37] Gallo-Oller G, Vollmann-Zwerenz A, Meléndez B, Rey JA, Hau P, Dotor J, et al. P144, a Transforming Growth Factor beta inhibitor peptide, generates antitumoral effects and modifies SMAD7 and SKI levels in human glioblastoma cell lines. Cancer letters. 2016;381:67-75.
[38] Huang C-Y, Chung C-L, Hu T-H, Chen J-J, Liu P-F, Chen C-L. Recent progress in TGF-β inhibitors for cancer therapy. Biomedicine & Pharmacotherapy. 2021;134:111046.

## Summary of the Invention

An object of the present invention is to provide the novel use of verbenone derivatives for treating and preventing the obesity-induced VAT inflammation and subsequent NAFLD by inhibiting the TGF-β/Smad3 signaling pathway in high-fat diet (HFD)-induced obesity mice.

To achieve the above object, the present invention provides a pharmaceutical composition for treating obesity comprising a verbenone derivative represented by Formula 1 below or a pharmaceutically acceptable salt thereof, as an active ingredient:

[Formula 1]

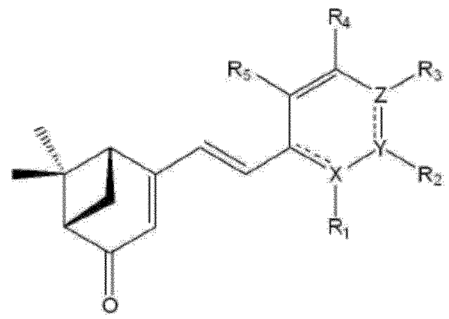

wherein $R_1$, $R_2$, $R_3$, $R_4$, and $R_5$ are each independently a hydrogen atom, a halogen atom, a hydroxyl group, a $C_{1-3}$ alkyl group, a $C_{1-3}$ alkoxy group, an amino group, a $C_{1-3}$ alkylamine group, a $C_{1-3}$ alkyldiamine group, a $C_{5-8}$ aryl group, a $C_{5-8}$ cyclic group, a $C_{5-8}$ heteroaryl group,

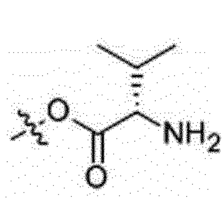, or 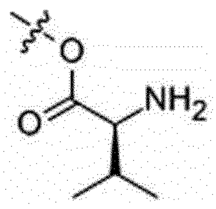;

X, Y and Z are each independently a carbon atom or an N, O or S atom; and

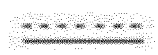

denotes a double bond or a single bond.

The present invention also provides a pharmaceutical composition for treating or preventing obesity-related liver disease and containing an active ingredient the verbenone derivative represented by Formula 1 or a pharmaceutically

acceptable salt thereof.

The present invention also provides a health functional food for preventing or ameliorating obesity comprising a verbenone derivative represented by Formula 1 below or a pharmaceutically acceptable salt thereof, as an active ingredient.

The present invention also provides a health functional food for preventing or ameliorating obesity-related liver disease comprising a verbenone derivative represented by Formula 1 below or a pharmaceutically acceptable salt thereof, as an active ingredient.

## Brief Description of Drawings

FIG. 1(A) shows a schematic diagram of the effect of SP-1154 on the TGF-β/Smad3 signaling pathway according to one embodiment of the present invention. TGF, transforming growth factor; VAT, visceral adipose tissue. (B and C) SP-1154 inhibits TGF-β1-stimualted complex formation of TGF-βR1 and TGF-βR2 in HEK293 cells. TGF-βR1, TGF-β type 1 receptor; TGF-βR2, TGF-β type 2 receptor. Data are presented as mean ± standard deviation (SD) of three independent experiments. Statistical significance was assessed by one-way analysis of variance (ANOVA) followed by post-hoc Tukey's test. $^{**}p < 0.01$ vs. TGF-β1-stimualted group without SP-1154 treatment. (D and E) SP-1154 inhibits phosphorylation of Smad2/3 in VAT of high fat diet (HFD)-induced mice. pSmad2, phosphorylated Smad2; pSmad3, phosphorylated Smad3. Data are presented as mean ± SD of three independent experiments. Statistical significance was assessed by one-way ANOVA followed by post-hoc Tukey's test. $^{*}p < 0.05$ *vs.* Normal. $^{***}p < 0.001$ vs. Normal. $^{\#}p < 0.05$ *vs.* HFD. $^{\#\#}p < 0.01$ *vs.* HFD. Expression mRNA levels of TGF-β1 (F), TGF-βR1 (G), and TGF-βR2 (H) of VAT in each group. GAPDH, glyceraldehyde 3-phosphate dehydrogenase. Data are presented as mean ± SD of four independent experiments. Statistical significance was assessed by one-way ANOVA followed by post-hoc Tukey's test. $^{*}p < 0.05$ *vs.* Normal. $^{**}p < 0.01$ *vs.* Normal.

FIG. 2 shows changes in body weight (A) and food intake (B and C) during the 20-week study period ($n = 5$ for normal, $n = 10$ for HFD, and $n = 8$ for HFD + SP-1154). Data are presented as mean ± SD. Statistical significance was assessed by one-way ANOVA followed by post-hoc Tukey's test. $^{*}p < 0.05$ vs. Normal. $^{**}p < 0.01$ *vs.* Normal. $^{***}p < 0.001$ *vs.* Normal. $^{\#\#}p < 0.01$ vs. HFD + SP-1154. $^{\#\#\#}p < 0.001$ vs. HFD + SP-1154.

FIG. 3 shows that SP-1154 reduced VAT SUVmax (A and B) and CRP level (C) in HFD-induced mice. (A and B) CT, computed tomography; PET, positron-emission tomography; ROI, region of interest; R, right; L, left; SUVmax, maximum standardized uptake value. $n = 6$ for normal, $n = 6$ for HFD, and $n = 6$ for HFD + SP-1154. Data are presented as mean ± SD. Statistical significance was assessed by one-way ANOVA followed by post-hoc Tukey's test. $^{***}p < 0.001$ *vs.* Normal. $^{\#}p < 0.05$ vs HFD. (C) CRP, C-reactive protein. $n = 10$ for normal, $n = 20$ for HFD, and $n = 15$ for HFD + SP-1154. Data are presented as mean ± SD. Statistical significance was assessed by one-way ANOVA followed by post-hoc Tukey's test. $^{***}p < 0.001$ vs. Normal. $^{\#\#\#}p < 0.001$ vs. HFD.

FIG. 4 shows that SP-1154 prevents macrophage infiltration into VAT of HFD-induced mice. (A) Representative $CD68^+$ immunostaining images of cross-sectional VATs. Nuclei were counterstained with 4',6-diamidino-2-phenylindole (DAPI). Scale bar, 100 μm (magnification, × 100). Representative flow cytometry plot charts of $F4/80^+CD11b^+$ macrophages in VAT of Normal (B), HFD (C), and HFD + SP-1154 (D). (E) Quantification of macrophages in VAT by flow cytometry in VAT of each group ($n = 4$ for normal, $n = 4$ for HFD, and $n = 4$ for HFD + SP-1154). Data are presented as mean ± SD. Statistical significance was assessed by one-way ANOVA followed by post-hoc Tukey's test. $^{***}p < 0.001$ vs. Normal. $^{\#\#}p < 0.01$ *vs.* HFD.

FIG. 5 shows that SP-1154 improves insulin resistance and glucose homeostasis. (A) Representative $GLUT1^+$ and $GLUT4^+$ immunostaining images of cross-sectional VATs (H&E, hematoxylin and eosin; GLUT, glucose transporter). Nuclei were counterstained with DAPI. Scale bar, 100 μm (magnification, × 100). Changes in insulin tolerance test (ITT) (B) and glucose tolerance test (GTT) (C) results ($n = 5$ for normal, $n = 10$ for HFD, and $n = 8$ for HFD + SP-1154). Data are presented as mean ± SD. Statistical significance was assessed by Kruskal-Wallis test with post-hoc Conover test. $^{**}p < 0.01$ *vs.* Normal. $^{\#\#}p < 0.01$ *vs.* HFD. (D) Quantification of serum glucose levels. $n = 5$ for normal, $n = 9$ for HFD, and $n = 8$ for HFD + SP-1154. Data are presented as mean ± SD. Statistical significance was assessed by one-way ANOVA followed by post-hoc Tukey's test. (E) Quantification of adipocyte surface area. $n = 4$ for normal, $n = 4$ for HFD, and $n = 4$ for HFD + SP-1154. Data are presented as mean ± SD. Statistical significance was assessed by one-way ANOVA followed by post-hoc Tukey's test. $^{***}p < 0.001$ vs. Normal. $^{\#}p < 0.05$ *vs.* HFD. Expression mRNA levels of GLUT1 (F) and GLUT4 (G). $n = 4$ for normal, $n = 4$ for HFD, and $n = 4$ for HFD + SP-1154. Data are presented as mean ± SD. Statistical significance was assessed by one-way ANOVA followed by post-hoc Tukey's test. $^{*}p < 0.05$ *vs.* Normal. $^{**}p < 0.01$ *vs.* Normal. $^{\#}p < 0.05$ vs. HFD. $^{\#\#}p < 0.01$ *vs.* HFD.

FIG. 6 shows that SP-1154 inhibits HFD-induced liver steatosis. (A) Representative images of liver CT and cross-sectional liver tissue (scale bar, 100 μm; magnification, × 100). Yellow arrows on coronal CT images indicate the

level of corresponding transverse CT images. (B) Quantification of liver density from CT images. *n* = 6 for normal, *n* = 6 for HFD, and *n* = 6 for HFD + SP-1154. Data are presented as mean ± SD. Statistical significance was evaluated by one-way ANOVA followed by post-hoc Tukey's test. $^{***}p < 0.001$ *vs.* Normal. $^{\#\#}p < 0.01$ vs. HFD. Quantification of liver weight (C) and liver steatosis score (D) from harvested liver. *n* = 5 for normal, *n* = 9 for HFD, and *n* = 8 for HFD + SP-1154. Data are presented as mean ± SD. Statistical significance was evaluated by one-way ANOVA followed by post-hoc Tukey's test. $^{**}p < 0.01$ vs. Normal. $^{\#\#}p < 0.01$ *vs.* HFD. Changes in serum AST, ALT (E) and total cholesterol (G) in response to SP-114 treatment. AST, aspartate aminotransferase; ALT, alanine aminotransferase. *n* = 5 for normal, *n* = 10 for HFD, and *n* = 8 for HFD + SP-1154. Data are presented as mean ± SD. Statistical significance was assessed by one-way ANOVA followed by post-hoc Tukey's test. $^{**}p < 0.01$ *vs.* Normal. $^{***}p < 0.001$ *vs.* Normal. $^{\#\#\#}p < 0.001$ *vs.* HFD.

## Detailed Description and Preferred Embodiments of the Invention

Unless otherwise defined, all technical and scientific terms used in the present specification have the same meanings as commonly understood by those skilled in the art to which the present invention pertains. In general, the nomenclature used in the present specification is well known and commonly used in the art.

In the present invention, it has been found that, in high-fat diet-induced mice, SP-1154 inhibits the TGF-β/Smad3 signaling pathway, remarkably suppresses high-fat diet-induced visceral adipose tissue (VAT) inflammation and its related systemic inflammation, significantly improves insulin sensitivity with glucose homeostasis, and reduces hepatic steatosis, thereby significantly ameliorates VAT inflammation and obesity-related NAFLD. This supports the potential use of SP-1154 as a therapeutic drug for obesity and its related NAFLD by targeting the inflamed VAT.

Therefore, the present invention is directed to a pharmaceutical composition for treating obesity comprising a verbenone derivative represented by Formula 1 below or a pharmaceutically acceptable salt thereof, as an active ingredient:

[Formula 1]

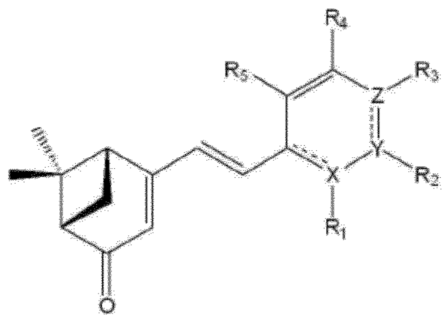

wherein $R_1$, $R_2$, $R_3$, $R_4$, and $R_5$ are each independently a hydrogen atom, a halogen atom, a hydroxyl group, a $C_{1-3}$ alkyl group, a $C_{1-3}$ alkoxy group, an amino group, a $C_{1-3}$ alkylamine group, a $C_{1-3}$ alkyldiamine group, a $C_{5-8}$ aryl group, a $C_{5-8}$ cyclic group, a $C_{5-8}$ heteroaryl group,

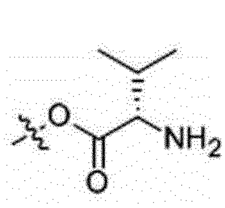 , or 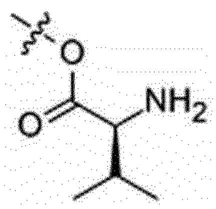 ;

X, Y and Z are each independently a carbon atom or an N, O or S atom; and

- - - - - - - denotes a double bond or a single bond.

Obesity-induced inflamed visceral adipose tissue (VAT) secretes pro-inflammatory cytokines, thereby promoting systemic inflammation and insulin resistance, which further exacerbate obesity-related non-alcoholic fatty liver disease (NAFLD), and TGF-β/Smad3 signaling plays an important role in inflammatory events in VAT. SP-1154 exhibits therapeutic effects against obesity-induced inflamed VAT and subsequent NAFLD by inhibiting TGF-β/Smad3 signaling in

high-fat diet-induced mice.

In the present invention, NAFLD was induced by a high-fat diet (60% fat) for 20 weeks using male C57BL/6 mice. SP-1154 (50 mg/kg) was orally administered daily for 20 weeks. *In vivo* VAT and systemic inflammation were measured by using $^{18}$F-fluorodeoxyglucose positron emission tomography and C-reactive protein levels. Both insulin tolerance test and glucose tolerance test were performed to assess the status of insulin resistance and glucose intolerance. Histological and molecular analyses were performed on harvested liver and VAT.

As a result, it was found that SP-1154 inhibited the TGF-β/Smad3 signaling pathway and remarkably suppressed high-fat diet-induced VAT inflammation and its related systemic inflammation. Furthermore, SP-1154 significantly improved insulin sensitivity with glucose homeostasis and reduced hepatic steatosis. SP-1154 significantly inhibited VAT inflammation and obesity-related NAFLD. In conclusion, it can be said that SP-1154 significantly ameliorates VAT inflammation and obesity-related NAFLD. This supports the potential use of SP-1154 as a therapeutic drug for obesity and its related NAFLD by targeting the inflamed VAT.

In order to better dissolve in water, SP-1154 was synthesized as a prodrug of the anti-inflammatory/antioxidant verbenone derivative SP-8356 [13]. Because the TGF-β/Smad3 signaling pathway is involved in both inflammation and oxidative stress [10], it was hypothesized that TGF-β/Smad3 signaling might be affected by SP-1154.

In the present invention, $R_1$, $R_2$, $R_3$, $R_4$, and $R_5$ may be each independently a hydrogen atom, a halogen atom, a hydroxyl group, a methyl group, an ethyl group, a methoxy group, an ethoxy group, an amino group, a $C_{5-6}$ aryl group, a $C_{5-6}$ cyclic group, a $C_{5-6}$ heteroaryl group,

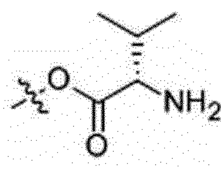
, or
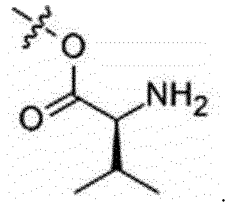
.

In the present invention, $R_1$, $R_2$, $R_3$, $R_4$, and $R_5$ may be independently a hydrogen atom, a halogen atom, a hydroxyl group, a methyl group, a methoxy group, a phenyl group, a pyrrole group, a pyridine group,

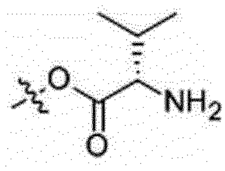
, or
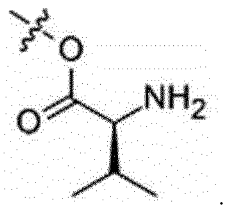
.

In the present invention, the verbenone derivative may be at least one selected from the group consisting of:

(1*S*,5*R*)-4-(4-hydroxystyryl)-6,6-dimethylbicyclo[3.1.1]hept-3-en-2-one (3a);
(1*S*,5*R*)-4-(4-hydroxy-2-methoxystyryl)-6,6-dimethylbicyclo[3.1.1]hept-3-en-2-one (3b);
(1*S*,5*R*)-4-(3,4-dihydroxystyryl)-6,6-dimethylbicyclo[3.1.1]hept-3-en-2-one (3c);
(1*S*,5*R*)-4-(3-bromo-4-hydroxystyryl)-6,6-dimethylbicyclo[3.1.1]hept-3-en-2-one (3d);
(1*S*,*SR*)-4-(4-hydroxy-2,6-dimethoxystyryl)-6,6-dimethylbicyclo[3.1.1] hept-3-en-2-one (3e);
(1*S*,5*R*)-4-(3,4-dihydroxy-5-methoxystyryl)-6,6-dimethylbicyclo[3.1.1]hept-3-en-2-one ( 3f);
(1*S*,5*R*)-4-(3-hydroxystyryl)-6,6-dimethylbicyclo[3.1.1]hept-3-en-2-one (3g);
(1*S*,5*R*)-4-(2-hydroxystyryl)-6,6-dimethylbicyclo[3.1.1]hept-3-en-2-one (3h);
(1*S*,5*R*)-4-(2-hydroxy-4-methoxystyryl)-6,6-dimethylbicyclo[3.1.1]hept-3-en-2-one (3i);
(1*S*,5*R*)-6,6-dimethyl-4-styrylbicyclo[3.1.1]hept-3-en-2-one (4a);
(1*S*,5*R*)-4-(4-fluorostyryl)-6,6-dimethylbicyclo[3.1.1]hept-3-en-2-one (4b);
(1*S*,5*R*)-4-(4-methoxystyryl)-6,6-dimethylbicyclo[3.1.1]hept-3-en-2-one (4c);
(1*S*,5*R*)-4-(2-(biphenyl-4-yl)vinyl)-6,6-dimethylbicyclo[3.1.1]hept-3-en-2-one (4d);
(1*S*,5*R*)-4-(4-(1H-pyrrol-1-yl)styryl)-dimethylbicyclo[3.1.1]hept-3-en-2-one (4e);
(1*S*,5*R*)-4-(3,4-dimethoxystyryl)-6,6-dimethylbicyclo[3.1.1]hept-3-en-2-one (4f);
(1*S*,5*R*)-4-(3,5-dimethoxystyryl)-6,6-dimethylbicyclo[3.1.1]hept-3-en-2-one (4g);
(1*S*,5*R*)-4-(2,5-dimethoxystyryl)-6,6-dimethylbicyclo[3.1.1]hept-3-en-2-one (4h);
(1*S*,5*R*)-4-(5-bromo-2-methoxystyryl)-6,6-dimethylbicyclo[3.1.1]hept-3-en-2-one (4i);

(1*S*,5*R*)-6,6-dimethyl-4-((E)-2-(pyridin-2-yl)vinyl)bicyclo[3.1.1]hept-3-en-2-one (5a);
(1*S*,5*R*)-6,6-dimethyl-4-((E)-2-(pyridin-3-yl)vinyl)bicyclo[3.1.1]hept-3-en-2-one (5b);
(1*S*,5*R*)-6,6-dimethyl-4-((E)-2-(pyridin-4-yl)vinyl)bicyclo[3.1.1]hept-3-en-2-one (5c); and
(2*S*,2'*S*)-5-((E)-2-((1*R*,5*S*)-6,6-dimethyl-4-oxobicyclo[3.1.1]hept-2-en-2-yl)vinyl)-3-methoxy-1,2-phenylene bis(2-amino-3-methylbutanoate)-dihydrochloride (6). Preferably, the verbenone derivative may be (1S,5R)-4-(3,4-dihydroxy-5-methoxystyryl)-6,6-dimethylbicyclo[3.1.1]hept-3-en-2-one (3f), or (2*S*,2'*S*)-5-((E)-2-((1R,5S)-6,6-dimethyl-4-oxobicyclo[3.1.1]hept-2-en-2-yl)vinyl)-3-methoxy-1,2-phenylene bis(2-amino-3-methylbutanoate)-dihydrochloride (6).

[Formula 2]

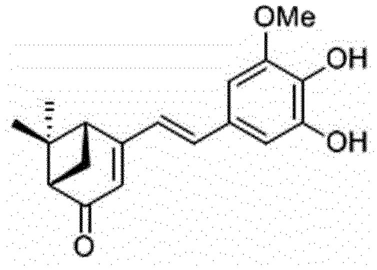

(Compound 3f)

[Formula 3]

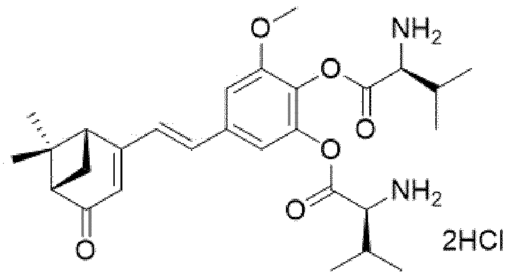

(Compound 6)

In the present invention, the obesity-related liver disease may be non-alcoholic fatty liver disease (NAFLD), wherein the fatty liver disease may be steatosis, steatohepatitis, non-alcoholic steatohepatitis (NASH), liver fibrosis, or cirrhosis.

In the present invention, the composition is able to reduce obesity-induced visceral adipose tissue inflammation by inhibiting the TGF-β/Smad3 signaling pathway.

In the present invention, SP-8356 does not dissolve well in saline, and thus when it is used as a suspension, the efficacy thereof is weak but significant. In addition, a valine ester of SP-8356 (SP-1154), a prodrug form of SP-8356, is converted to SP-8356 very quickly and has high water solubility, and thus it is used after dissolution in viscous hyaluronic acid. Soluble SP-8356 (SP-1154) is more effective than SP-8356 administered as a suspension.

As used herein, the term "treatment" refers to any action that alleviates or beneficially alters symptoms of obesity or obesity-related liver disease by administration of the composition comprising a verbenone derivative represented by Formula 1 below or a pharmaceutically acceptable salt thereof, as an active ingredient according to the present invention.

As used herein, the term "prevention" refers to any action that suppresses or delays symptoms of obesity or obesity-related liver disease by administration of the composition comprising a verbenone derivative represented by Formula 1 below or a pharmaceutically acceptable salt thereof, as an active ingredient according to the present invention.

As used herein, the term "prodrug" refers to a derivative of a drug molecule that requires transformation to the parent drug by enzymes or hydrolysis to release the active drug inside and outside the body. Prodrugs are frequently, although not necessarily, pharmacologically inactive until converted into the parent drug. Prodrugs are frequently, but

not necessarily, pharmaceutically inactive while the parent drug is being converted.

As used herein, the term "non-alcoholic fatty liver disease (NAFLD)" refers to fatty liver disease that occurs due to causes other than alcohol. The term "non-alcoholic fatty liver disease" encompasses "non-alcoholic steatosis" and "non-alcoholic steatohepatitis", which are conditions in which fat accumulates in hepatocytes due to causes other than alcohol, "non-alcoholic fatty liver-associated fibrosis", and "non-alcoholic fatty liver-associated cirrhosis".

As used herein, the term "fatty liver" refers to a case where there is fat deposition within the liver but no hepatocellular injury (ballooning degeneration) and fibrosis. "Steatohepatitis" refers to a case where there are inflammatory findings accompanied by hepatocellular injury (balloon degeneration) while showing fat deposition within the liver. Steatohepatitis may also be accompanied by liver fibrosis. "Cirrhosis" refers to cirrhosis accompanied by histological findings of fatty liver or steatohepatitis, or cirrhosis that occurred in patients who previously had histologically proven fatty liver or steatohepatitis. In the present specification, the definitions of terms related to fatty liver disease are only intended to include various conditions related to fatty liver, and the conditions of patients defined according to the terms cannot always be clearly distinguished.

As used herein, the term "dyslipidemia" refers to hyperlipidemia and hypercholesterolemia. Hyperlipidemia and hypercholesterolemia each refer to a condition in which plasma triglyceride levels are higher than those in plasma of normal people and plasma total cholesterol and/or LDL-cholesterol levels are higher.

The compounds represented by Formula 1 according to the present invention may be prepared as pharmaceutically acceptable salts or solvates according to conventional methods known in the art. A pharmaceutically acceptable salt is advantageously an acid addition salt formed with a pharmaceutically acceptable free acid. An acid addition salt may be prepared using a conventional method, for example, by dissolving a compound in an excess amount of aqueous acid solution and precipitating the resulting salt using a water-miscible organic solvent, such as methanol, ethanol, acetone, or acetonitrile. Alternatively, an acid addition salt may be prepared by heating an equimolar amount of a compound and an acid in water or an alcohol (e.g., glycol monomethyl ether), and then drying the mixture by evaporation or filtering the precipitated salt by suction.

In this case, an inorganic acid or an organic acid may be used as the free acid. Examples of the inorganic acids include hydrochloric acid, phosphoric acid, sulfuric acid, nitric acid, stannic acid, and the like, and examples of the organic acids include methanesulfonic acid, p-toluenesulfonic acid, acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like.

In addition, a pharmaceutically acceptable metal salt may be prepared using a base. An alkali metal or alkaline earth metal salt may be obtained, for example, by dissolving a compound in an excess amount of alkali metal hydroxide or alkaline earth metal hydroxide solution, filtering undissolved salt, and then drying the filtrate by evaporation. In this case, as the metal salts, in particular, sodium, potassium or calcium salts are pharmaceutically suitable. Also, the corresponding silver salts may be obtained by reacting an alkali metal or alkaline earth metal salt with a proper silver salt (e.g., silver nitrate).

Unless otherwise indicated, pharmaceutically acceptable salts of the compounds having the structure of Formula 1 include salts of acidic or basic groups, which may be present in the compounds having the structure of Formula 1. For example, pharmaceutically acceptable salts include sodium, calcium and potassium salts of a hydroxyl group, and other pharmaceutically acceptable salts of an amino group, including hydrobromide, sulfate, hydrogen sulfate, phosphate, hydrogen phosphate, dihydrogen phosphate, acetate, succinate, citrate, tartrate, lactate, mandelate, methanesulfonate (mesylate), and para-toluenesulfonate (tosylate). The salts may be prepared using a salt preparation method or preparation process known in the art.

As used herein, the term "pharmaceutical composition" refers to one prepared for the purpose of preventing or treating disease, and each composition may be formulated in various forms according to conventional methods and used. For example, the pharmaceutical composition may be formulated in dosage forms, such as powders, granules, tablets, capsules, suspensions, emulsions, and syrups, and may be formulated in dosage forms, such as external preparations, suppositories, and sterile injectable solutions. Specifically, the pharmaceutical composition may be formulated and used in forms suitable for administration by instillation, for example, eye drops, creams, ointments, gels, or lotions.

The routes of administration of the pharmaceutical composition according to the present invention include, but are not limited to, oral, intravenous, intramuscular, intraarterial, intramedullary, intrathecal, intracardiac, transdermal, subcutaneous, intraperitoneal, intranasal, gastrointestinal, topical, sublingual, instillation, or rectal routes. As used herein, the term "parenteral" includes subcutaneous, intradermal, intravenous, intramuscular, intra-articular, intra-synovial, intrasternal, intrathecal, intralesional and intracranial injection or infusion techniques. The pharmaceutical composition of the present invention may also be administered in the form of suppositories for rectal administration. The pharmaceutical composition of the present invention may be orally administered in any orally acceptable dosage forms, including, but not limited to, capsules, tablets, and aqueous suspensions and solutions. In the case of tablets for oral use, carriers

which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, sweetening, flavoring and/or coloring agents may be added.

The pharmaceutical composition of the present invention may vary depending upon a variety of factors, including the activity of the specific compound used, the age, body weight, general health status, sex, diet, time of administration, route of administration, rate of excretion, drug combination, and the severity of the specific disease to be prevented or treated.

In the present invention, the pharmaceutical composition may be formulated with or used in combination with one or more agents selected from the group consisting of calcium channel blockers, antioxidants, glutamate antagonists, anticoagulants, antihypertensive agents, antithrombotic agents, antihistamine agents, anti-inflammatory analgesics, anticancer agents, and antibiotics.

In another aspect, the present invention is directed to a health functional food for preventing or ameliorating obesity or obesity-related liver disease comprising a verbenone derivative represented by Formula 1 below or a pharmaceutically acceptable salt thereof, as an active ingredient.

The functional food of the present invention may be used in various applications, including drugs, foods or beverages for the prevention of inflammation. Examples of the functional food of the present invention include various foods, candies, chocolates, beverages, gums, teas, vitamin complexes, health supplement foods, and the like, and the functional food may be used in the forms of powders, granules, tablets, capsules or beverages.

The composition containing the compound according to the present invention may be formulated in the form of oral preparations such as powders, granules, capsules, tablets, and aqueous suspensions, preparations for external use, suppositories, and sterile injectable solutions, according to respective conventional methods, and used. Carriers, excipients and diluents that may be contained in the composition include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, magnesium stearate, and mineral oil. The composition is formulated using diluents or excipients, such as fillers, extenders, binders, wetting agents, disintegrants, surfactants, etc., which are commonly used. Solid formulations for oral administration include tablets, pills, powders, granules, capsules, etc. Such solid formulations are prepared by mixing the compound with at least one excipient, such as cotton, starch, calcium carbonate, sucrose, lactose, gelatin, etc. In addition to simple expedients, lubricants such as magnesium stearate, talc, etc. may also be used. Liquid formulations for oral administration include suspensions, oral solutions, emulsions, syrups, etc., which may comprise commonly used simple diluents, e.g., water and liquid paraffin, as well as various excipients, e.g., wetting agents, sweeteners, flavoring agents, preservatives, etc. Formulations for parenteral administration include sterile aqueous solutions, non-aqueous solvents, suspensions, emulsions, lyophilized formulations, suppositories, etc. As non-aqueous solvents and suspending agents, propylene glycol, polyethylene glycol, vegetable oils such as olive oil, injectable esters such as ethyl oleate, etc. may be used. As a base for suppositories, Witepsol, Macrogol, Tween 61, cacao fat, laurin fat, glycerogelatin, etc. may be used.

Although the preferred dosage of the compound of the present invention varies depending on the patient's condition and body weight, the severity of disease, the type of drug, and the route and duration of administration, it may be suitably selected by a person skilled in the art. In order to achieve the desired effects, however, the compound may be administered at a daily dose of 0.01 mg/kg to 10 g/kg, preferably 1 mg/kg to 1 g/kg. The compound may be administered once or several times a day. Therefore, the dosage is not intended to limit the scope of the present invention in any way.

In another aspect, the present invention is directed to a method for treating or preventing obesity or obesity-related liver disease comprising a step of administering, to a subject, a pharmaceutical composition for treating or preventing obesity or obesity-related liver disease containing, as an active ingredient, the verbenone represented by Formula 1 or a pharmaceutically acceptable salt thereof. (USA)

In another aspect, the present invention is directed to the novel use of a pharmaceutical composition for treating or preventing obesity or obesity-related liver disease containing, as an active ingredient, the verbenone represented by Formula 1 or a pharmaceutically acceptable salt thereof. (Europe/China)

Hereinafter, the present invention will be described in more detail with reference to examples. However, these examples are only for illustrating the present invention, and it will be obvious to those skilled in the art that the scope of the present invention is not limited by these examples.

**[EXAMPLES]**

**[Examples]**

**Production Example 1: Production of Verbenone Derivatives**

Reagent grade (1*S*)-(-)-verbenone, 3,4-dihydroxy-5-methoxybenzaldehyde, chloromethyl methyl ether (MOM-Cl), diisopropylethylamine (DIPEA), potassium hydroxide (KOH), and sodium methoxide ($NaOCH_3$) were commercially purchased. All the purchased reagents and solvents had high purity, and were used directly without additional purification, except for dichloromethane distilled with calcium hydroxide. Unless specified otherwise, reaction was performed in vacuum-flame dried glassware under a dry nitrogen atmosphere. Thin-layer chromatography (TLC) was performed using Merck silica gel 60 $F_{254}$ that is visualized by UV light, and column chromatography was performed using silica gel (E. Merck silica gel, 70-230, 230-400 mesh).

$^1$H-NMR and $^{13}$C-NMR spectra were measured using an instrument (Varian) at 500 MHz, and chemical shifts were reported in ppm from tetramethylsilane (TMS) as an internal standard ($CDCl_3$: d 7.26 ppm), and coupling constant was recorded in Hertz. Multiplicity was recorded using the following abbreviations: singlet (s), doublet (d), doublet of doublet (dd), doublet of doublet of doublet (ddd), triplet (t), triplet of doublet (td), doublet of triplet (dt), quartet (q), multiplet (m), and broad (br). Fetal bovine serum (FBS) was purchased from a company (Hyclone, Logan, Utah), and neurobasal medium (NBM) and B27 supplement were purchased from a company (Invitrogen, Carlsbad, Calif.). All chemicals and reagents were purchased from a company (Sigma-Aldrich, St. Louis, Mo.).

The compound of Formula 3f was produced according to Reaction Scheme 1 below:

[Reaction Scheme 1]

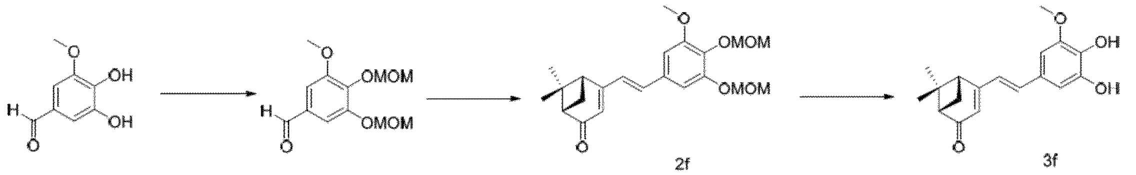

2f

3f

**Production of (1*S*,5*R*)-4-(3-methoxy-4,5-bis(methoxymethoxy)styryl)-6,6-dimethylbicyclo[3.1.1]hept-3-en-2-one (2f) compound**

400 mg of 3,4-dihydroxy-5-methoxybenzaldehyde was added to 4 mL of dichloromethane, and then 920 mg of diisopropylethylamine was added thereto, followed by cooling. 570 mg of methylchloromethyl ether was added thereto and stirred at room temperature. After adding a small amount of water thereto and separating into layers, the organic layer was dehydrated and concentrated under reduced pressure.

In order to obtain diene from (1S)-(-)-verbenone by aldol condensation, 380 mg of (1S)-(-)-verbenone 1 and 620 mg of 3-methoxy-4,5-bis(methoxymethoxy)benzaldehyde were added to 6.2 mL of MeOH and 270 mg of KOH was added thereto, followed by stirring at 60°C and cooling to room temperature. After adding a small amount of water thereto, the organic layer was separated, dehydrated, and concentrated under reduced pressure to obtain a yellow product which was then purified by silica gel column chromatography to obtain (1S,SR)-4-(3-methoxy-4,5-bis(methoxymethoxy)styryl)-6,6-dimethylbicyclo[3.1.1]hept-3 -en-2-one (2f) (90%yield).

$^1$H-NMR ($CDCl_3$, 500MHz);
*d*6.94 (d, *J*=1.96Hz, 1H), 6.84 (s, 2H), 6.78 (d, *J*=1.71Hz, 1H), 5.92 (s, 1H), 5.22 (s, 2H), 5.15 (s, 2H), 3.89 (s, 3H), 3.60 (s, 3H), 3.52 (s, 3H), 3.09 (t, *J*=5.75 Hz, 1H), 2.90 (dt, *J*=9.48, 5.53Hz, 1H), 2.72 (td, *J*=5.75, 1.47Hz, 1H), 2.10 (d, *J*=9.29Hz, 1H), 1.57 (s, 3H), 1.00(s, 3H);
$^{13}$C-NMR ($CDCl_3$, 75MHz); *d*203.92, 164.02, 153.63, 151.19, 136.64, 134.76, 132.10, 126.91, 122.43, 108.91, 104.88, 98.37, 95.33, 58.19, 57.11, 56.07, 52.70, 43.78, 39.93, 26.70, 22.11.

**Production of (1S,5R)-4-(3,4-dihydroxy-5-methoxystyryl)-6,6-dimethylbicyclo[3.1.1]hept-3-en-2-one) (3f) compound**

960 mg of compound 2f was added to 5 mL of MeOH, and 630 mg of concentrated hydrochloric acid was added

dropwise thereto, followed by stirring. The reaction solution was made neutral by adding saturated $NaHCO_3$, extracted with ethyl acetate, dehydrated, and then concentrated under reduced pressure. The resulting compound was purified and separated by column chromatography to obtain (1S,5R)-4-(3,4-dihydroxy-5-methoxystyryl)-6,6-dimethylbicyclo[3.1.1]hept-3-en-2-one (3f) (92% yield) as a yellow solid showing the following physical property values, and the obtained compound was used as a sample in the following Experimental Example:

mp 168-170°C;
$[a]^{20}{}_{D}$-158.8000° (c1.0, MeOH);
$^1$H-NMR ($CDCl_3$, 500MHz) *d*6.78-6.82 (m,3H), 6.64 (d, *J*=1.47Hz, 1H), 5.82-6.01 (m, 3H), 3.92 (s, 3H), 3.10 (t, *J*=5.62Hz, 1H), 2.91 (dt, *J*=9.35, 5.59Hz, 1H), 2.74 (td, *J*=5.69, 1.59Hz, 1H), 2.12 (d, *J*=9.29Hz, 1H), 2.04 (s, 2H), 1.58 (s,3H),1.01 (s, 3H);
$^{13}$C-NMR($CDCl_3$, 75MHz); d204.92, 165.07, 147.23, 144.24, 135.54, 134.13, 128.07, 125. 50, 121.62, 108.63, 58.08, 56.25, 53.12, 43.75, 40.18, 26.78, 22.16;
HRMS: calculated for $C_{18}H_{20}O_4$ (M+H) 301.1440, found 301.1453;
HPLC analytical result: (method 1) 100% ($t_R$=3.46 min).

Prodrug SP-1154 was produced according to Reaction Scheme 2 below:

[Reaction Scheme 2]

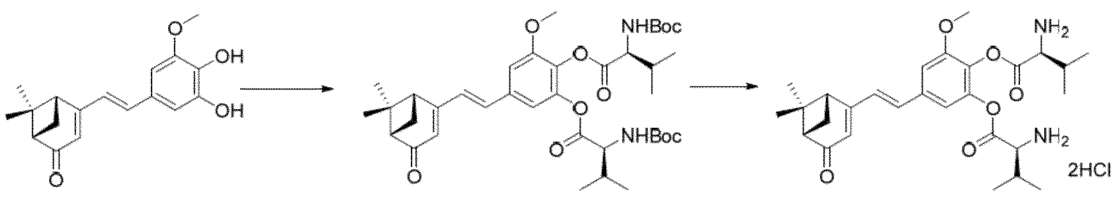

3f
SP-8356

LTV

SP-1154

**Production LTV of (2S,2'S)-5-((E)-2-((1R,5S)-6,6-dimethyl-4-oxobicyclo[3.1.1]hept-2-en-2-yl)vinyl)-3-methoxy-1,2-phenylene bis(2-((tert-butoxycarbonynamino)-3-methytbutanoate) compound**

To a stirred solution of compound 3f (SP-8356, (1S,SR)-4-(3,4-dihydroxy-5-methoxystyryl)-6,6-dimethylbicyclo[3.1.1]hept-3-en-2-one, 600 mg, 2.0 mmol) in dichloromethane (5 mL), N-(tert-butoxycarbonyl)-L-valine (Boc-Val-OH (1.3g 6.0 mmol)), 1-ethyl-3-(3-dimethylaminopropyl)carbodiimide hydrochloric acid (EDCI, 1.15 g, 6.0 mmol) and 4-dimethylaminopyridine (DMAP, 120 mg, 1.0 mmol) were added, followed by stirring at room temperature for 1 hour. After completion of the reaction, 6 mL of water was added and the organic layer was separated and then washed with 6 mL of 20% NaCl aqueous solution. The organic layer was dehydrated with anhydrous sodium sulfate and concentrated under reduced pressure. Recrystallization from 6 mL of diethyl ether gave (2*S*,2'*S*)-5-((*E*)-2-((1*R*,5*S*)-6,6-dimethyl-4-oxobicyclo[3.1.1]hept-2-en-2-yl)vinyl)-3-methoxy-1,2-phenylene bis(2-((tert-butoxycarbonyl)amino)-3-methylbutanoate) (67% yield).

$^1$H-NMR ($CDCl_3$, 400 MHz): *d* 6.97 (2H, brs), 6.90 (1H, d, *J* = 16.0 Hz), 6.84 (1H, d, *J* = 15.5 Hz), 5.95 (1H, s), 5.34 (1H, d, *J* = 9.0 Hz, NH), 5.14 (1H, d, *J* = 9.0 Hz, NH), 4.23-4.50 (2H, m), 3.87 (3H, s), 3.09 (1H, t, *J* = 5.5 Hz), 2.91-2.95 (1H, m), 2.73-2.75 (1H, m), 2.32-2.40 (2H, m), 2.12 (1H, d, *J* = 9.5 Hz), 2.04 (3H, s), 1.59 (3H, s), 1.47 (18H, brs), 1.01-1.09 (12H, m)
$^{13}$C-NMR ($CDCl_3$, 100 MHz): *d* 204.0, 169.9, 169.4, 163.6, 155.9, 155.6, 152.4, 143.5, 134.7, 133.5, 132.0, 128.5, 123.3, 114.3, 108.0, 80.0, 79.9, 59.1, 58.4, 58.2, 56.1, 52.9, 43.6, 39.9, 31.1, 30.8, 28.3, 26.7, 22.1, 19.2, 19.1, 17.7, 17.1

**Production of (2*S*,2'*S*)-5-((*E*)-2-((1*R*,5*S*)-6,6-dimethyl-4-oxobicyclo[3.1.1]hept-2-en-2-yl)vinyl)-3-methoxy-1,2-phenylene bis(2-amino-3-methylbutanoate) dihydrochloride compound**

9.2 mL of acetonitrile and 9.2 mL of 2N HCl diethyl ether solution were added to (2*S*,2'*S*)-5-((*E*)-2-((1*R*,5*S*)-6,6-dimethyl-4-oxobicyclo[3.1.1]hept-2-en-2-yl)vinyl)-3-methoxy-1,2-phenylene bis(2-((tert-butoxycarbonyl)amino)-3-methylbutanoate) (920 mg, 1.32 mmol). After stirring at room temperature for 3 hours, the solid was filtered, and 18.4 mL of ethyl acetate was added thereto, followed by stirring for 24 hours. The solid was filtered and dried to obtain (2*S*,2'*S*)-5-((*E*)-2-((1*R*,5*S*)-6,6-dimethyl-4-oxobicyclo[3.1.1]hept-2-en-2-yl)vinyl)-3-methoxy-1,2-phenylene bis(2-amino-3-methylbutanoate) dihydrochloride (89% yield).

$^1$H-NMR (DMSO-$d_6$, 400 MHz): *d* 9.00 (6H, brs), 7.48 (1H, m), 7.33 (1H, d, *J* = 16.0 Hz), 7.19 - 7.24 (2H, m), 5.96 (1H, s), 4.52 (2H, m), 3.88 (3H, s), 3.23 (1H, t, *J* = 5.0 Hz), 2.92 (1H, m), 2.58 (1H, d, *J* = 4.5 Hz), 2.38 - 2.43 (2H, m), 1.96 (1H, d, *J* = 9.0 Hz), 1.54 (3H, s), 1.08 - 1.14 (12H, m), 0.91 (3H, s)
$^{13}$C-NMR ($CDCl_3$, 100 MHz): *d* 203.1, 167.2, 166.8, 164.6, 152.3, 142.7, 136.2, 134.0, 131.0, 129.6, 123.13, 123.08, 114.6, 109.8, 58.0, 57.4, 57.35, 57.0, 56.9, 52.5, 43.2, 30.0, 26.7, 22.3, 18.9, 18.3, 18.2, 17.7.

## Example 1: Effect of Verbenone Derivatives against NAFLD

### 1.1. Synthesis of SP-1154: Structural Complementation Assay

Molecular interaction between TGF-βR1 and TGF-βR2 was investigated using NanoBiT technology established by Promega (Madison, WI, USA) as described in previous study [14]. In brief, HEK293 cells were seeded into 96-well microplates at a density of 2.0 × $10^4$ cells/ well. The next day, 50 ng of each plasmid containing a TGF-βR1-LgBiT or TGF-βR2-SmBiT construct was introduced into the cells using Lipofectamine 2000 (Invitrogen, Carlsbad, CA, USA). After 24 hours, before measuring luminescence, the cells were stabilized for 10 min at room temperature by replacing the media with 100 μl of Opti-MEM. Then, 25 μl Nano-Glo Live Cell Reagent (furimazine) from Promega was added to each well, and baseline luminescence was measured for the first 10 min. In this step, SP1154 was added to some wells at indicated concentration. Finally, the cells were stimulated using 10 μl of TGF-β1 at a concentration of 10 ng/ mL, and cell-plate measurements were continued for 1 hour. These procedures were conducted using a luminometer (BioTek Instruments, Inc., Winooski, VT, USA).

### 1.2. Induction of NAFLD

Male C57BL/6 mice (6 weeks old, 20 g body weight) were purchased from Orient-Bio (Seongnam, Korea). All mice were maintained in a 12-hr light/dark cycle and fed *ad libitum.* To minimize environmental differences, mice were allowed 2 weeks for acclimation before the start of experiments. For diet-induced NAFLD experiments, mice were fed either an HFD (60% of kcal in fat, D12492, Research Diets, NJ, USA) or a normal chow diet (ND) for 20 weeks. All experimental protocols and procedures were approved by the Ethics Committee and the Institutional Animal Care and Use Committee of Korea University College of Medicine (Approval No. KOREA-2019-0026).

### 1.3. Drug Treatment

The mice were subdivided into vehicle and SP-1154 (50 mg/kg as SP-8356) groups. SP-1154 was administrated to the mice via tab water and the dose of drug was determined by daily water consumption (6 mL/day) [15] and body weights, which were measured every week.

### 1.4. $^{18}$F-Fluorodeoxyglucose Positron Emission Tomography/Computed Tomography ($^{18}$F-FDG PET/CT) Imaging

$^{18}$F-FDG PET/CT was performed using a dedicated small animal PET/CT scanner (eXplore Vista DR PET/CT, GE Healthcare, Milwaukee, WI, USA). Mice were not fasted before taking examination and anesthetized with 2% isoflurane in oxygen (1 L/min) during the examination. PET/CT image acquisition was started 40 min after the injection of 18.5 MBq/0.1 mL of $^{18}$F-FDG via the tail vein. Static PET images were acquired for 20 min, followed by CT scan (40 kV, 250 μA). Image reconstruction was performed using Fourier rebinning with the ordered subsets expectation maximization (OSEM) algorithm with decay, attenuation, random, and normalization correction. The voxel size and axial slice thickness were 0.3875 × 0.3875 mm and 0.775 mm, respectively. After image reconstruction, coronal-, transverse-, sagittal view, and maximum intensity projection images were generated.
Images were analyzed by two experienced nuclear medicine physicians (KP and HWK) using commercially available software (Syngo. via, Siemens Healthcare, Erlangen, Germany). Metabolic activity of VAT assessed by $^{18}$F-FDG PET/CT has been shown to reflect the inflammatory status of VAT in human studies [16, 17]. For the evaluation of VAT metabolic activity, region on interest (ROI) was placed along the intra-abdominal fat on each slice and the maximum standardized uptake value (SUVmax) was measured, as previously described [16, 17]. Averaged VAT SUVmax from those ROIs was used for analysis. SUV was calculated as follows:

$$SUV = Tracer\ activity\ (ROI)\ (MBq/mL)/$$

$$Injected\ dose\ (MBq)/Total\ body\ weight\ (g)$$

$$SUV = Tracer\ activity\ (ROI)\ (MBq/mL)/$$

$$Injected\ dose\ (MBq)/Total\ body\ weight\ (g)$$

Next, for the measurement of Hounsfield units (HU) of the liver, ROI was drawn over the liver on each slice and the corresponding HU value was acquired. Averaged HU values from those ROIs were used as representative HU values. The intra- and interobserver correlation coefficient values of VAT SUV max, and liver HU on targeted ROIs were > 0.9.

**1.5. Enzyme-Linked Immunosorbent Assay (ELISA)**

Serum concentration of C-reactive protein (CRP) was determined using a commercially available ELISA kit (ab157712, Abcam, Cambridge, MA, USA) according to the manufacturer's instruction.

**1.6. Insulin Tolerance Test (ITT) and Glucose Tolerance Test (GTT)**

For GTT, glucose (2 g/kg body weight) was orally administered to mice after overnight fasting, and blood was drawn to measure glucose concentrations at indicated times thereafter. For ITT, insulin (1.5 insulin units/kg body weight) was intraperitoneally injected into mice after fasting for 4 hours, and blood was drawn to measure blood glucose at indicated times. Insulin concentrations were measured at each indicated time in serum collected from the mice subjected to GTT.

**1.7. Blood Analysis**

Blood samples were collected immediately before sacrifice. The levels of AST (aspartate aminotransferase), ALT (alanine aminotransferase), total cholesterol, and triglyceride in serum were measured using a FUJI DRI-Chemiclinical Chemistry Analyzer (FUJI DRI-CHEM 4000i, Fuji Film, Tokyo, Japan).

**1.8. Flow Cytometry Analysis**

Cells were suspended in 0.1 mL of fluorescence-activated cell sorting (FACS) buffer (phosphate-buffered saline; PBS with 5% fetal bovine serum; FBS) and incubated with Fc blocker (BD Bioscience, San Jose, CA, USA) at room temperature for 10 min, followed by an additional 30 min with anti-CD45 APC-Cy7, anti-F4/80 PE, and anti-CD11b-PerCP-Cy5.5 (Biolegend, San Diego, CA, USA) antibodies according to the manufacturer's recommendation. After staining, flow cytometry was performed using FACS Canto II (BD Bioscience, San Jose, CA, USA). Data were collected using FACSDiva software (BD Bioscience, San Jose, CA, USA) and analyzed using FlowJo software (FlowJo LLC, Ashland, OR, USA).

**1.9. Histopathology**

Mice were sacrificed after 20 weeks of drug administration by using the $CO_2$ chamber. Fresh liver and VAT harvested from the sacrificed mice were fixed with 4% paraformaldehyde (PFA) for 12 hours at 4°C and washed with PBS for overnight at 4 °C. Paraffin-embedded tissues were cut into 5 $\mu$m sections and were stained with hematoxylin and eosin (H&E) for morphology analysis. Adipocyte surface area of a minimum of 300 cells per group was measured using ImageJ software (version 1.45 s, Bethesda, MD, USA), as previously described. Hepatic steatosis was assessed using three-scale scoring system based on H&E-stained liver: score 1 (mild, 5-33%); score 2 (moderate, 34-66%); score 3 (severe, > 66%) [19]. For Oil Red O staining, mice livers were fixed with 4% PFA and cryoprotected in 30% sucrose solution. Next, liver tissues were embedded in optimal cutting temperature compound (Scigen Scientific, Gardena, CA, USA) and cut into 8 $\mu$m sections with a cryostat microtome (Leica CM 3050S, Leica Microsystems, Wetzlar, Germany).

### 1.10. Immunohistochemistry

Tissues were incubated with anti-CD68 (ab125212, Abcam, Cambridge, MA, USA), anti-glucose transporter 1 (GLUT1) (ab652, Abcam, Cambridge, MA, USA), and anti-GLUT4 (ab654, Abcam, MA, USA). Alexa Fluor 594 (Invitrogen, Carlsbad, CA, USA) was used as secondary antibody. All fluorescence images were acquired with Zeiss Axio Scan.Z1 (Carl Zeiss, Jena, Germany).

### 1.11. Quantitative Real-Time RT-PCR (qRT-PCR)

Total RNA was extracted from VAT samples using the Trizol reagent (Invitrogen, Carlsbad, CA, USA) and the generation of cDNA was performed using the reverse transcription reaction kit (iScript cDNA synthesis kit, Bio-Rad, Hercules, CA, USA). Gene expression was assessed using SYBR Green mixture (iQTM SYBR Green Supermix, Bio-Rad, Hercules, CA, USA) and an iCycler PCR thermocycler (Bio-Rad, Hercules, CA, USA). Target gene mRNA template-specific primer sets were designed from GenScript (Piscataway, NJ, USA) and the levels of mRNA were normalized with the mRNA level of glyceraldehyde 3-phosphate dehydrogenase (GAPDH) [20]. The primer sequences used in qRT-PCR were as follows: GAPDH (5'-ATGTGTCCGTCGTGGATCTG-3' and 5'-AAGTCGCAGGACAACCTG-3'), GLUT1 (5'-AGTATTGGAGCAACTGTGC-3' and 5'-GCCTTTGGTCTCAGGGACTT-3'), GLUT4 (5'-GCCATCGTCATTGGCATTCT-3' and 5'-GGAGAGCAGGGAGTACTGTG-3'), TGF-β1 (5'-CACTCCCGTGGCTTCTAGTG-3' and 5'-GGACTGGCGAGCCTTAGTTT-3'), TGF-βR1 (5'-TTGCACTTG) ' and 5'-AGCTGCCAGCTCCACAGGAC-3'), and TGF-βR2 (5'-CACAGTGTGTGGCAGAGC-3' and 5'-GTTCAGCGAGCCATCTTCTG-3').

### 1.12. Western Blot Analysis

VAT was lysed in RIPA buffer (Thermo Fisher Scientific, Waltham, MA, USA) with protease inhibitor (GeneDepot, Barker, TX, USA). The protein concentration was quantified using the BCA protein assay kit (Thermo Fisher Scientific, Waltham, MA, USA). Next, proteins were separated by sodium dodecyl sulfate polyacrylamide gel electrophoresis gel and transferred to polyvinylidene fluoride membrane (PVDF, Merck Millipore, Billerica, MA, USA). The membranes were incubated with Smad2/3 (sc-133098, Santa Cruz Biotechnology, Santa Cruz, CA, USA), phospho-Smad2(3108S, Cell Signaling Technology, Danvers, MA, USA) and phospho-Smad3 (9520S, Cell Signaling Technology, Danvers, MA, USA). Protein bands were detected using the ECL assay kit (GE Healthcare, Chicago, IL, USA).

### 1.13. Statistical Analysis

All data were expressed as mean ± standard deviation. Shapiro-Wilk test was performed to determine normality distribution. One-way analysis of variance (ANOVA) followed by post-hoc Tukey's test was used for parametric analysis and Kruskal-Wallis test with post-hoc Conover test was used for non-parametric analysis. Data were analyzed using MedCalc version 18.5 (MedCalc, Mariakerke, Belgium) and SPSS version 17.0 (SPSS Inc., Chicago, IL, USA). A $p$-value of $< 0.05$ was considered statistically significant.

### Effect of SP-1154 on Inhibition of TGF-β/Smad3 Signaling Pathway

To determine the effect of SP-1154 on the interaction of TGF-βR1 with TGF-βR2, the present inventors performed a structural complementation assay using NanoBiT technology with the vectors containing the genes of both receptors conjugated with the Nano Luciferase fragments. A complex of the large fragment (LgBiT) and the small fragment (SmBit) of Nano Luciferase generates a bright luminescent signal, thereby make it possible to study protein-protein interactions [14]. As shown in FIGS. 1B and 1C, TGF-β1 increased the luciferase activities from the complex of TGF-βR1 and TGF-βR2 and treatment with SP-1154 significantly reduced the TGF-β1-stimualted activities in a dose-dependent manner. Next, the present inventors examined the effect of SP-1154 on the phosphorylation of Smad2/3, which is a downstream effector of TGF-β, in VAT of HFD-induced mice. Notably, phosphorylation of Smad2/3 was upregulated in the HFD group, and that was inhibited by SP-1154 treatment (FIGS. 1D and 1E). Regarding the mRNA expression levels of TGF-β1, TGF-βR1 and TGF-βR2 in VAT, there was no significant difference between HFD and HFD with SP-1154 groups (FIGS. 1F to 1H). Accordingly, SP-1154 inhibits the interaction of TGF-βR1 with TGF-βR2, thereby suppressing the phosphorylation of Smad2/3.

### Effects of SP-1154 on Inhibition of HFD-induced Body Weight Gain

As can be seen in FIG. 2A, SP-1154 significantly attenuated the HFD-induced body weight gain. Moreover, there was no significant difference between HFD and HFD with SP-1154 groups in terms of food consumption (FIGS.

2B and 2C). Thus, SP-1154 inhibits HFD-induced body weight gain without affecting appetite.

**Effects of SP-1154 on Reduction of VAT Inflammation and Its Related Systemic Inflammation**

The present inventors first investigated whether VAT inflammation was attenuated by SP-1154, by using $^{18}$F-FDG PET/CT, which is a well-known non-invasive imaging modality for the assessment of VAT inflammation, mainly produced by macrophages [16, 17]. It was found that VAT SUVmax significantly increased after 20 weeks of HFD and SP-1154 significantly reduced the elevated VAT SUVmax (FIGS. 3A and 3B). There was no significant difference of VAT SUVmax between normal mice and SP-1154-treated mice. Consistently, the levels of CRP, a surrogate marker for systemic inflammation [17], increased in HFD group and were significantly suppressed by SP-1154 (FIG. 3C). There was also no significant difference of CRP levels between normal mice and SP-1154-treated mice.

Next, to confirm the results from the *in vivo* PET/CT, the present inventors performed IHC and flow cytometry analysis on harvested VAT. As shown in FIG. 4A, the expression of CD68, a macrophage marker, was upregulated in HFD mice and suppressed by SP-1154 treatment. Furthermore, flow cytometry analysis revealed that VAT from HFD mice showed increased macrophage infiltration compared to normal mice and SP-1154 treatment significantly prevented macrophage infiltration into VAT (FIGS. 4B to 4E). These results indicate that SP-1154 suppresses obesity-induced VAT inflammation and its related systemic inflammation.

**Effects of SP-1154 on improvement of Insulin Resistance and Glucose Homeostasis**

Since VAT inflammation contributes to the development of insulin resistance [1, 4, 6], the present inventors further evaluated the therapeutic effect of SP-1154 on insulin resistance. The present inventors first assessed the expressions of insulin-dependent GLUT4 and insulin-independent GLUT1 which are the hallmarks of insulin resistance in VAT. During insulin resistance in VAT, enlarged adipocytes show reduced expression of insulin dependent GLUT4, whereas inflammatory cells such as macrophages show increased expression of insulin-independent GLUT1 [21, 22]. Consistent with previous studies, it was found that 20 weeks of HFD increased the adipocyte size with an increase of GLUT1 expression and a reduction of GLUT4 expression. Notably, SP-1154 induced a significant reduction in adipocyte size with decreased GLUT1 expression and increased GLUT4 expression (FIGS. 5A, 5E,5 F, and 5G). Furthermore, consistently, SP-1154 significantly improved insulin resistance with glucose homeostasis in HFD-induced mice (FIGS. 5B and 5C). These results indicate that SP-1154 improves obesity-induced insulin resistance and glucose homeostasis.

**Effect of SP-1154 on Attenuation of HFD-Induced Liver Steatosis**

To investigate the effect of SP-1154 on HFD-induced liver steatosis, the present inventors first performed CT scan to measure liver density. Liver density, measured in HU, is a surrogate marker of fat infiltration with lower HU reflecting more fat infiltration [23]. In the present invention, it was found that the live density was lowered in the HFD group and significantly enhanced in the SP-1154 group (FIGS. 6A and B). Similarly, both liver weight and liver steatosis scores increased in the HFD group and were significantly reduced by SP-1154 treatment (FIGS. 6A, 6C, and 6D). SP-1154 also improved the levels of AST and ALT, which were both elevated in HFD-induced mice (FIGS. 6E and 6F). There was no significant difference between HFD and HFD with SP-1154 groups in terms of serum total cholesterol (FIG. 6G). Altogether, these results indicate that SP-1154 inhibits obesity-related NAFLD in HFD-induced mice.

Visceral obesity has been recognized as a low-grade, chronic, systemic inflammation, which contributes to the development of NAFLD [1, 4-6]. Discovering approaches to reduce obesity-related VAT inflammation thereby preventing the progression of NAFLD could have a major clinical impact. In this sense, the present inventors clearly identified that SP-1154, a novel synthetic drug, effectively suppressed VAT inflammation, thereby attenuating obesity-related NAFLD.

HFD-induced mice model was used to evaluate the therapeutic efficacy of SP-1154. Feeding mice with HFD has been well known to induce obesity, insulin resistance, and hepatic steatosis, thereby closely mimicking the metabolically unhealthy obesity phenotype of humans [24, 25], which are also consistent with the results of the Examples of the present invention. Thus, the present invention also highlights the potential applicability of SP-1154 for extrapolation of data to clinical fields.

Macrophages play key roles in the development of obesity-related VAT inflammation [7]. In normal VAT, macrophages approximately account for less than 15% of the whole cell population [26]. However, in obesity, macrophages infiltrate into VAT and account for up to 50% of the cell population in VAT with increased pro-inflammatory cytokines secretion [26]. It was found that SP-1154 significantly suppressed both HFD-induced macrophage infiltration into VAT and its related systemic inflammation. Furthermore, this is the first animal study to investigate the anti-inflammatory effect of pharmaceutical intervention on obesity-related VAT inflammation using $^{18}$F-FDG PET/CT, an established non-invasive imaging modality for evaluating VAT inflammation in human beings [16, 17].

Interestingly, infiltrated macrophages in VAT also block insulin action in adipocyte via downregulation of GLUT4

in adipocyte membrane thereby contributing to the development of insulin resistance [27, 28], which were consistent with the results of the Examples of the present invention. The present inventors characterized a novel drug, SP-1154, that effectively improves insulin resistance and restores GLUT4 expression in the inflamed VAT. Thus, the collective findings support that the beneficial metabolic effect of SP-1154 could be attributed to its therapeutic effect on VAT inflammation, mainly on macrophages.

Currently, most new drug development efforts against NAFLD are focused on advanced stages of NAFLD such as nonalcoholic steatohepatitis and stage 2-3 liver fibrosis or cirrhosis [29]. However, the therapeutic strategy for early-stage NAFLD is also clinically beneficial in patients with NAFLD [30]. Patients with early-stage NAFLD have potentially more easily reversible liver injury, whereas they have higher chance of developing end-stage liver disease with excess cardiometabolic risk [30, 31]. Thus, SP-1154 could be a promising therapeutic drug for early-stage NAFLD by attenuating hepatic steatosis and preventing the progression of NAFLD.

The results of the Examples of the present invention show that SP-1154 suppresses the formation of a complex of TGF-βR1 and TGF-βR2, thereby inhibiting Smad3 phosphorylation. As the interaction between TGF-β1 and the complex of TGF-βR1 and TGF-βR2 is a key initiating factor in the TGF-β/Smad3 signaling pathway [10], monoclonal neutralizing antibodies targeting TGF-β have shown inhibitory effects on TGF-β/Smad3 signaling in preclinical studies [12, 32]. Furthermore, similar to monoclonal antibodies, Fc-fusion protein targeting TGF-β receptor also suppresses TGF-β/Smad3 signaling in both cell and animal models [33]. However, these protein engineering-based approaches have several limitations that continue to impede their clinical application, such as unfavorable pharmacokinetics, unclear mode of action, high production cost, incorrect protein conformation, and low protein yield [34, 35]. In contrast, SP-1154, like other small-molecular drugs, is easy to manufacture, is inexpensive, and provides the great advantage of oral bioavailability.

Similar to SP-1154, a synthetic peptide P144 derived from human betaglycan, is also known to inhibit the formation of a complex of TGF-βR1 and TGF-βR2, thereby exhibiting anti-fibrotic and anti-tumor effects in preclinical studies [36, 37]. However, due to its toxicity, P144 has been approved only for topical use in clinical trials for systemic sclerosis patients with skin fibrosis (NCT00574613 and NCT 00781053) [38]. To the best knowledge of the present inventors, SP-1154 is the first new synthetic drug developed to treat obesity-related metabolic disease by preventing the formation of a complex of TGF-βR1 and TGF-βR2. However, further studies are needed to fully elucidate the detailed underlying mechanism of action of SP-1154 on the interaction between TGF-βR1 and TGF-βR2, including the actual binding site.

In summary, the present inventors provided evidence that SP-1154 significantly reduces obesity-induced VAT inflammation and attenuates subsequent hepatic steatosis in HFD-fed mice. SP-1154 improved VAT inflammation by reducing macrophage recruitment to VAT. The anti-inflammatory effect of SP-1154 in VAT appears to be mediated through inhibition of the TGF-β/Smad3 signaling pathway. Taken together, these findings suggest that SP-1154 may be a promising drug for the treatment of obesity and its related NAFLD.

## Industrial Applicability

The compound of Formula 1 according to the present invention is able to significantly reduce obesity-induced VAT inflammation and attenuate subsequent hepatic steatosis in HFD-fed mice, and is able to alleviate VAT inflammation by reducing macrophage recruitment to VAT.

In the present invention, the anti-inflammatory effect in VAT appears to be mediated through inhibition of the TGF-β/Smad3 signaling pathway, and this effect suggests that the compound of Formula 1 according to the present invention may be a promising drug for the treatment of obesity or obesity-related liver disease such as NAFLD.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A pharmaceutical composition for treating obesity comprising a verbenone derivative represented by Formula 1 below or a pharmaceutically acceptable salt thereof, as an active ingredient:

[Formula 1]

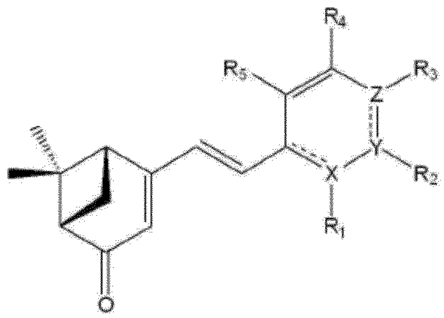

wherein

$R_1$, $R_2$, $R_3$, $R_4$ and $R_5$ are each independently a hydrogen atom, a halogen atom, a hydroxyl group, a $C_{1-3}$ alkyl group, a $C_{1-3}$ alkoxy group, an amino group, a $C_{1-3}$ alkylamine group, a $C_{1-3}$ alkyldiamine group, a $C_{5-8}$ aryl group, a $C_{5-8}$ cyclic group, a $C_{5-8}$ heteroaryl group,

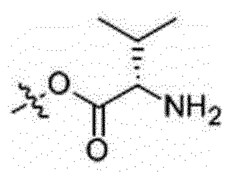, or 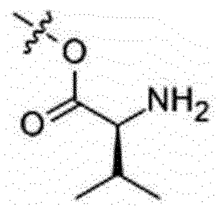;

X, Y and Z are each independently a carbon atom or an N, O or S atom; and

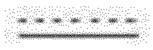

denotes a double bond or a single bond.

2. A pharmaceutical composition for treating obesity-related liver disease comprising a verbenone derivative represented by Formula 1 below or a pharmaceutically acceptable salt thereof, as an active ingredient:

[Formula 1]

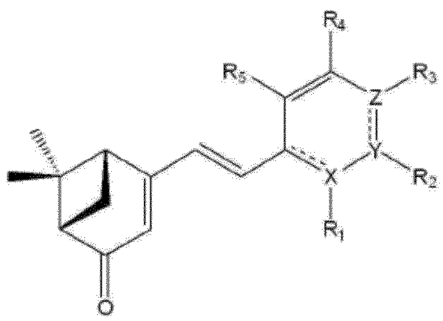

wherein

$R_1$, $R_2$, $R_3$, $R_4$, and $R_5$ are each independently a hydrogen atom, a halogen atom, a hydroxyl group, a $C_{1-3}$ alkyl group, a $C_{1-3}$ alkoxy group, an amino group, a $C_{1-3}$ alkylamine group, a $C_{1-3}$ alkyldiamine group, a $C_{5-8}$ aryl group, a $C_{5-8}$ cyclic group, a $C_{5-8}$ heteroaryl group,

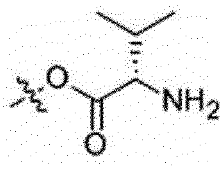, or 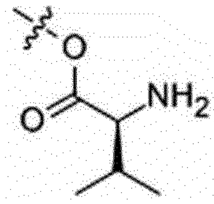;

X, Y and Z are each independently a carbon atom or an N, O or S atom; and

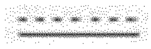

denotes a double bond or a single bond.

3. The pharmaceutical composition of claim 1 or 2, wherein $R_1$, $R_2$, $R_3$, $R_4$, and $R_5$ are each independently a hydrogen atom, a halogen atom, a hydroxyl group, a methyl group, an ethyl group, a methoxy group, an ethoxy group, an amino group, a $C_{5-6}$ aryl group, a $C_{5-6}$ cyclic group, a $C_{5-6}$ heteroaryl group,

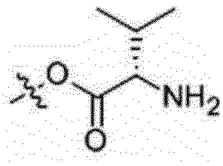,

or

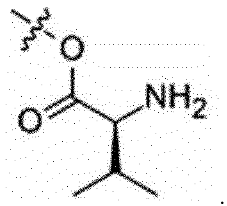.

4. The pharmaceutical composition of claim 3, wherein $R_1$, $R_2$, $R_3$, $R_4$, and $R_5$ are each independently a hydrogen atom, a halogen atom, a hydroxyl group, a methyl group, a methoxy group, a phenyl group, a pyrrole group, a pyridine group,

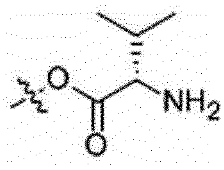, or 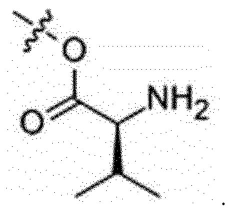.

5. The pharmaceutical composition of claim 1 or 2, wherein the verbenone derivative is at least one selected from the group consisting of:

(1*S*,5*R*)-4-(4-hydroxystyryl)-6,6-dimethylbicyclo[3.1.1]hept-3-en-2-one (3a);
(1*S*,5*R*)-4-(4-hydroxy-2-methoxystyryl)-6,6-dimethylbicyclo[3.1.1]hept-3-en-2-one (3b);
(1*S*,5*R*)-4-(3,4-dihydroxystyryl)-6,6-dimethylbicyclo[3.1.1]hept-3-en-2-one (3c);
(1*S*,5*R*)-4-(3-bromo-4-hydroxystyryl)-6,6-dimethylbicyclo[3.1.1]hept-3-en-2-one (3d);
(1*S*,5*R*)-4-(4-hydroxy-2,6-dimethoxystyryl)-6,6-dimethylbicyclo[3.1.1] hept-3-en-2-one (3e);
(1*S*,5*R*)-4-(3,4-dihydroxy-5-methoxystyryl)-6,6-dimethylbicyclo[3.1.1]hept-3-en-2-one ( 3f);
(1*S*,5*R*)-4-(3-hydroxystyryl)-6,6-dimethylbicyclo[3.1.1]hept-3-en-2-one (3g);
(1*S*,5*R*)-4-(2-hydroxystyryl)-6,6-dimethylbicyclo[3.1.1]hept-3-en-2-one (3h);

(1*S*,5*R*)-4-(2-hydroxy-4-methoxystyryl)-6,6-dimethylbicyclo[3.1.1]hept-3-en-2-one (3i);
(1*S*,5*R*)-6,6-dimethyl-4-styrylbicyclo[3.1.1]hept-3-en-2-one (4a);
(1*S*,5*R*)-4-(4-fluorostyryl)-6,6-dimethylbicyclo[3.1.1]hept-3-en-2-one (4b);
(1*S*,5*R*)-4-(4-methoxystyryl)-6,6-dimethylbicyclo[3.1.1]hept-3-en-2-one (4c);
(1*S*,5*R*)-4-(2-(biphenyl-4-yl)vinyl)-6,6-dimethylbicyclo[3.1.1]hept-3-en-2-one (4d);
(1*S*,5*R*)-4-(4-(1H-pyrrol-1-yl)styryl)-dimethylbicyclo[3.1.1]hept-3-en-2-one (4e);
(1*S*,5*R*)-4-(3,4-dimethoxystyryl)-6,6-dimethylbicyclo[3.1.1]hept-3-en-2-one (4f);
(1*S*,5*R*)-4-(3,5-dimethoxystyryl)-6,6-dimethylbicyclo[3.1.1]hept-3-en-2-one (4g);
(1*S*,5*R*)-4-(2,5-dimethoxystyryl)-6,6-dimethylbicyclo[3.1.1]hept-3-en-2-one (4h);
(1*S*,5*R*)-4-(5-bromo-2-methoxystyryl)-6,6-dimethylbicyclo[3.1.1]hept-3-en-2-one (4i);
(1*S*,5*R*)-6,6-dimethyl-4-((E)-2-(pyridin-2-yl)vinyl)bicyclo[3.1.1]hept-3-en-2-one (5a);
(1*S*,5*R*)-6,6-dimethyl-4-((E)-2-(pyridin-3-yl)vinyl)bicyclo[3.1.1]hept-3-en-2-one (5b);
(1*S*,5*R*)-6,6-dimethyl-4-((E)-2-(pyridin-4-yl)vinyl)bicyclo[3.1.1]hept-3-en-2-one (5c); and
(2*S*,2'*S*)-5-((E)-2-((1*R*,5*S*)-6,6-dimethyl-4-oxobicyclo[3.1.1]hept-2-en-2-yl)vinyl)-3-methoxy-1,2-phenylene bis(2-amino-3-methylbutanoate)-2-hydrochloride (6).

6. The pharmaceutical composition of claim 1 or 2, wherein the verbenone derivative is (1*S*,5*R*)-4-(3,4-dihydroxy-5-methoxystyryl)-6,6-dimethylbicyclo[3.1.1]hept-3-en-2-one (3f), or (2*S*,2'*S*)-5-((E)-2-((1R,5S)-6,6-dimethyl-4-oxobicyclo[3.1.1]hept-2-en-2-yl)vinyl)-3-methoxy-1,2-phenylene bis(2-amino-3-methylbutanoate)-2-hydrochloride (6).

7. The pharmaceutical composition of claim 2, wherein the obesity-related liver disease is non-alcoholic fatty liver disease (NAFLD).

8. The pharmaceutical composition of claim 7, wherein the fatty liver disease is steatosis, steatohepatitis, liver fibrosis, or cirrhosis.

9. The pharmaceutical composition of claim 1 or 2, wherein the composition reduces obesity-induced visceral adipose tissue inflammation by inhibiting TGF-β/Smad3 signaling pathway.

10. A health functional food for preventing or ameliorating obesity comprising a verbenone derivative represented by Formula 1 below or a pharmaceutically acceptable salt thereof, as an active ingredient:

[Formula 1]

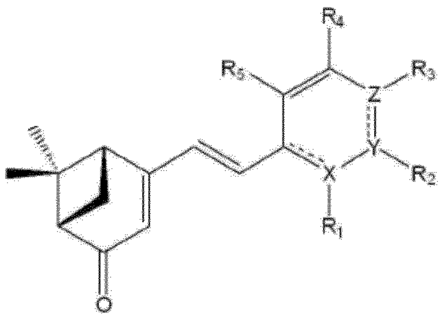

wherein

$R_1$, $R_2$, $R_3$, $R_4$, and $R_5$ are each independently a hydrogen atom, a halogen atom, a hydroxyl group, a $C_{1-3}$ alkyl group, a $C_{1-3}$ alkoxy group, an amino group, a $C_{1-3}$ alkylamine group, a $C_{1-3}$ alkyldiamine group, a $C_{5-8}$ aryl group, a $C_{5-8}$ cyclic group, a $C_{5-8}$ heteroaryl group,

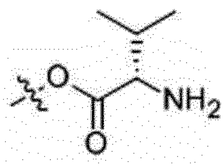, or 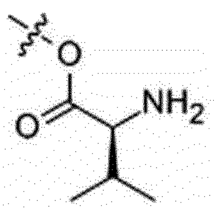;

X, Y and Z are each independently a carbon atom or an N, O or S atom; and

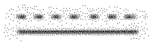

denotes a double bond or a single bond.

**11.** A health functional food for preventing or ameliorating obesity-related liver disease comprising a verbenone derivative represented by Formula 1 below or a pharmaceutically acceptable salt thereof, as an active ingredient:

[Formula 1]

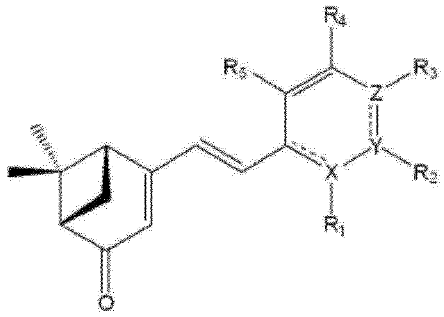

wherein

$R_1$, $R_2$, $R_3$, $R_4$, and $R_5$ are each independently a hydrogen atom, a halogen atom, a hydroxyl group, a $C_{1-3}$ alkyl group, a $C_{1-3}$ alkoxy group, an amino group, a $C_{1-3}$ alkylamine group, a $C_{1-3}$ alkyldiamine group, a $C_{5-8}$ aryl group, a $C_{5-8}$ cyclic group, a $C_{5-8}$ heteroaryl group,

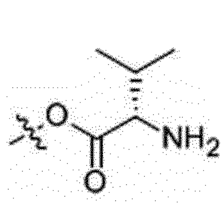, or 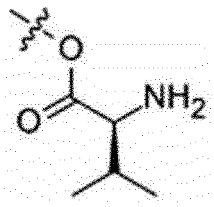;

X, Y and Z are each independently a carbon atom or an N, O or S atom; and

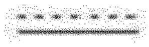

denotes a double bond or a single bond.

**12.** The health functional food of claim 10 or 11, wherein $R_1$, $R_2$, $R_3$, $R_4$, and $R_5$ are each independently a hydrogen atom, a halogen atom, a hydroxyl group, a methyl group, an ethyl group, a methoxy group, an ethoxy group, an amino group, a $C_{5-6}$ aryl group, a $C_{5-6}$ cyclic group, a $C_{5-6}$ heteroaryl group,

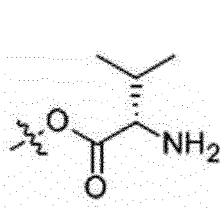, or 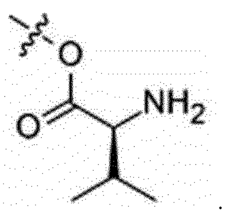.

**13.** The health functional food of claim 10 or 11, wherein $R_1$, $R_2$, $R_3$, $R_4$, and $R_5$ are each independently a hydrogen atom, a halogen atom, a hydroxyl group, a methyl group, a methoxy group, a phenyl group, a pyrrole group, a pyridine group,

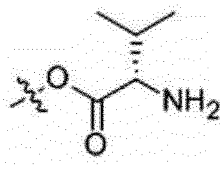

, or

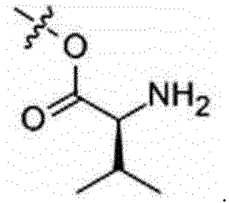

.

**14.** The health functional food of claim 10 or 11, wherein the verbenone derivative is at least one selected from the group consisting of:

(1*S*,5*R*)-4-(4-hydroxystyryl)-6,6-dimethylbicyclo[3.1.1]hept-3-en-2-one (3a);
(1*S*,5*R*)-4-(4-hydroxy-2-methoxystyryl)-6,6-dimethylbicyclo[3.1.1]hept-3-en-2-one (3b);
(1*S*,5*R*)-4-(3,4-dihydroxystyryl)-6,6-dimethylbicyclo[3.1.1]hept-3-en-2-one (3c);
(1*S*,5*R*)-4-(3-bromo-4-hydroxystyryl)-6,6-dimethylbicyclo[3.1.1]hept-3-en-2-one (3d);
(1*S*,5*R*)-4-(4-hydroxy-2,6-dimethoxystyryl)-6,6-dimethylbicyclo[3.1.1] hept-3-en-2-one (3e);
(1*S*,5*R*)-4-(3,4-dihydroxy-5-methoxystyryl)-6,6-dimethylbicyclo[3.1.1]hept-3-en-2-one ( 3f);
(1*S*,5*R*)-4-(3-hydroxystyryl)-6,6-dimethylbicyclo[3.1.1]hept-3-en-2-one (3g);
(1*S*,5*R*)-4-(2-hydroxystyryl)-6,6-dimethylbicyclo[3.1.1]hept-3-en-2-one (3h);
(1*S*,5*R*)-4-(2-hydroxy-4-methoxystyryl)-6,6-dimethylbicyclo[3.1.1]hept-3-en-2-one (3i);
(1*S*,5*R*)-6,6-dimethyl-4-styrylbicyclo[3.1.1]hept-3-en-2-one (4a);
(1*S*,5*R*)-4-(4-fluorostyryl)-6,6-dimethylbicyclo[3.1.1]hept-3-en-2-one (4b);
(1*S*,5*R*)-4-(4-methoxystyryl)-6,6-dimethylbicyclo[3.1.1]hept-3-en-2-one (4c);
(1*S*,5*R*)-4-(2-(biphenyl-4-yl)vinyl)-6,6-dimethylbicyclo[3.1.1]hept-3-en-2-one (4d);
(1*S*,5*R*)-4-(4-(1H-pyrrol-1-yl)styryl)-dimethylbicyclo[3.1.1]hept-3-en-2-one (4e);
(1*S*,5*R*)-4-(3,4-dimethoxystyryl)-6,6-dimethylbicyclo[3.1.1]hept-3-en-2-one (4f);
(1*S*,5*R*)-4-(3,5-dimethoxystyryl)-6,6-dimethylbicyclo[3.1.1]hept-3-en-2-one (4g);
(1*S*,5*R*)-4-(2,5-dimethoxystyryl)-6,6-dimethylbicyclo[3.1.1]hept-3-en-2-one (4h);
(1*S*,5*R*)-4-(5-bromo-2-methoxystyryl)-6,6-dimethylbicyclo[3.1.1]hept-3-en-2-one (4i);
(1*S*,5*R*)-6,6-dimethyl-4-((E)-2-(pyridin-2-yl)vinyl)bicyclo[3.1.1]hept-3-en-2-one (5a);
(1*S*,5*R*)-6,6-dimethyl-4-((E)-2-(pyridin-3-yl)vinyl)bicyclo[3.1.1]hept-3-en-2-one (5b);
(1*S*,5*R*)-6,6-dimethyl-4-((E)-2-(pyridin-4-yl)vinyl)bicyclo[3.1.1]hept-3-en-2-one (5c); and
(2*S*,2'*S*)-5-((E)-2-((1R,5S)-6,6-dimethyl-4-oxobicyclo[3.1.1]hept-2-en-2-yl)vinyl)-3-methoxy-1,2-phenylene bis(2-amino-3-methylbutanoate)-2-dihydrochloride (6).

**15.** The health functional food of claim 10 or 11, wherein the verbenone derivative is (1*S*,5*R*)-4-(3,4-dihydroxy-5-methoxystyryl)-6,6-dimethylbicyclo[3.1.1]hept-3-en-2-one (3f), or (2*S*,2'*S*)-5-((E)-2-((1R,5S)-6,6-dimethyl-4-oxobicyclo[3.1.1]hept-2-en-2-yl)vinyl)-3-methoxy-1,2-phenylene bis(2-amino-3-methylbutanoate)-2-dihydrochloride (6).

**16.** The health functional food of claim 11, wherein the obesity-related liver disease is non-alcoholic fatty liver disease (NAFLD).

**17.** The health functional food of claim 16, wherein the fatty liver disease is steatosis, steatohepatitis, liver fibrosis, or cirrhosis.

**FIG. 1**

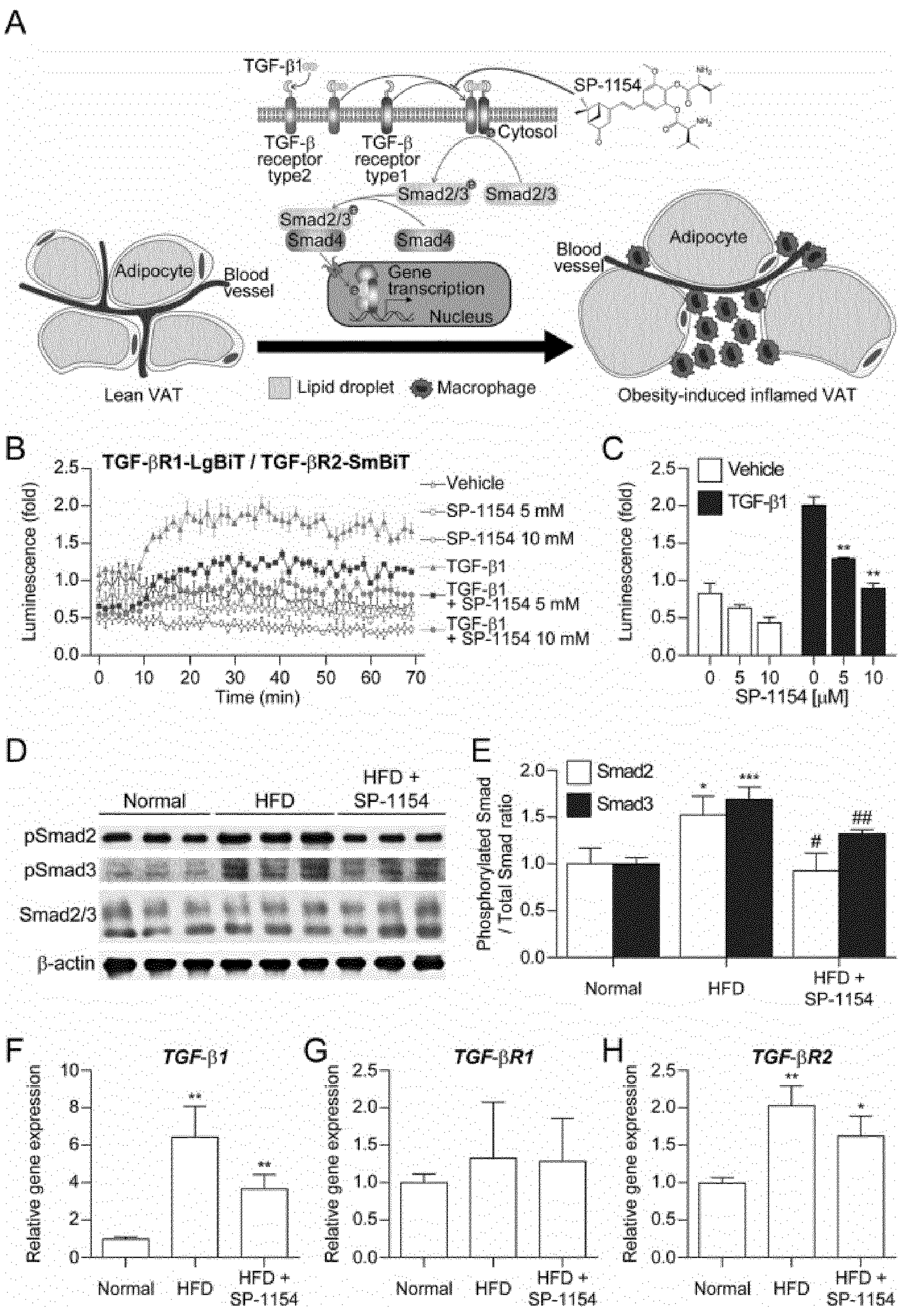
A
TGF-β1
SP-1154
Cytosol
TGF-β receptor type2
TGF-β receptor type1
Smad2/3
Smad4
Gene transcription
Nucleus
Adipocyte
Blood vessel
Lean VAT
Lipid droplet
Macrophage
Obesity-induced inflamed VAT
B
TGF-βR1-LgBiT / TGF-βR2-SmBiT
Luminescence (fold)
Time (min)
Vehicle
SP-1154 5 mM
SP-1154 10 mM
TGF-β1
TGF-β1 + SP-1154 5 mM
TGF-β1 + SP-1154 10 mM
C
Vehicle
TGF-β1
SP-1154 [μM]
D
Normal
HFD
HFD + SP-1154
pSmad2
pSmad3
Smad2/3
β-actin
E
Phosphorylated Smad / Total Smad ratio
Smad2
Smad3
F
TGF-β1
Relative gene expression
G
TGF-βR1
H
TGF-βR2

FIG. 2

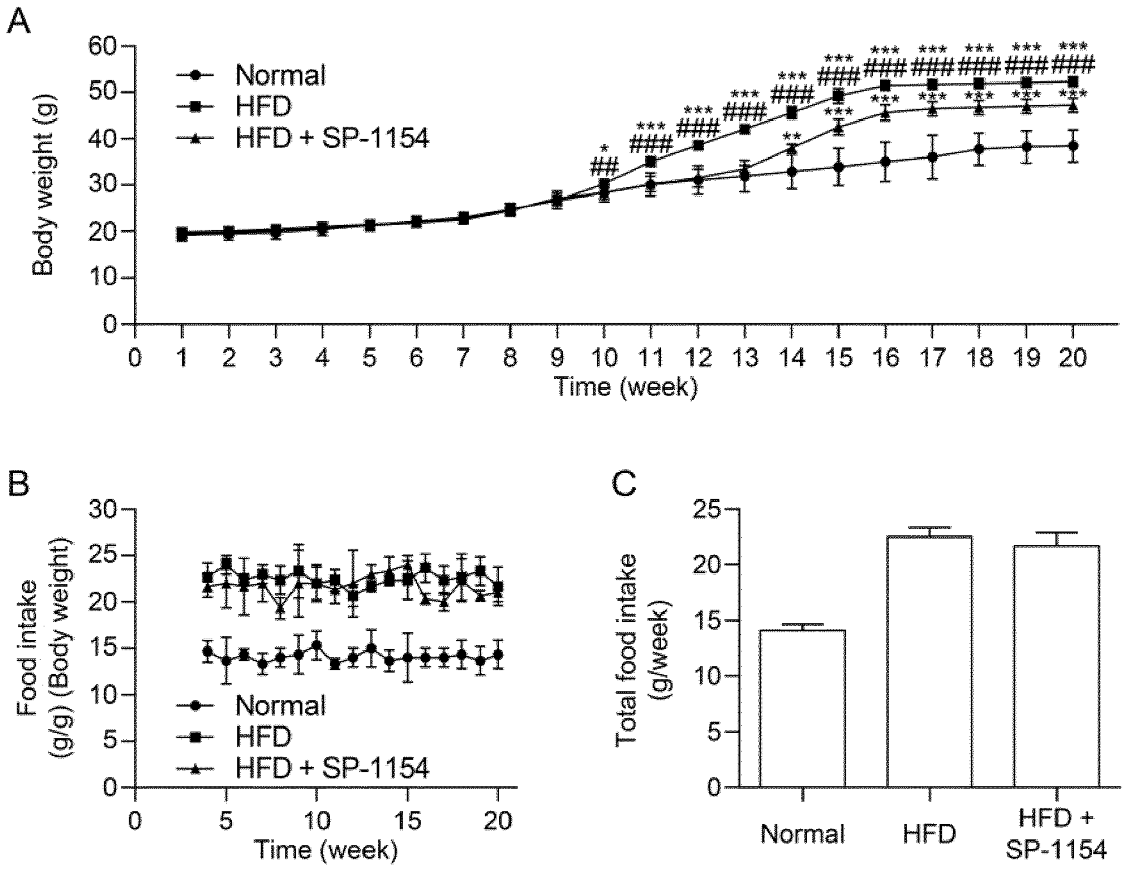
A
Body weight (g)
Time (week)
Normal
HFD
HFD + SP-1154
B
Food intake (g/g) (Body weight)
Time (week)
Normal
HFD
HFD + SP-1154
C
Total food intake (g/week)
Normal
HFD
HFD + SP-1154

FIG. 3

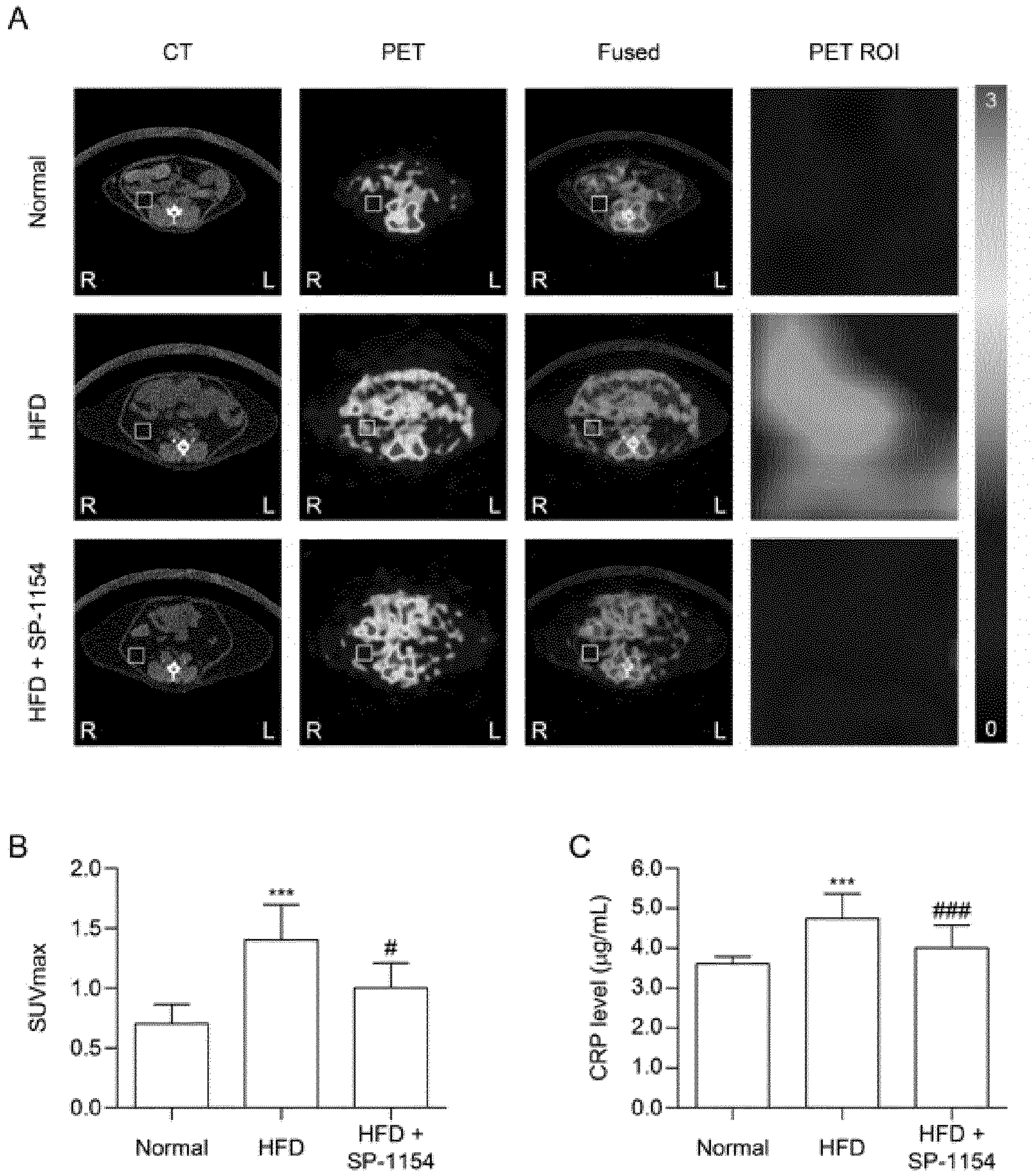
A
CT
PET
Fused
PET ROI
Normal
HFD
HFD + SP-1154
R
L
3
0
B
2.0
1.5
1.0
0.5
0.0
SUVmax
***
#
Normal
HFD
HFD + SP-1154
C
6.0
5.0
4.0
3.0
2.0
1.0
0.0
CRP level (μg/mL)
***
###
Normal
HFD
HFD + SP-1154

FIG. 4

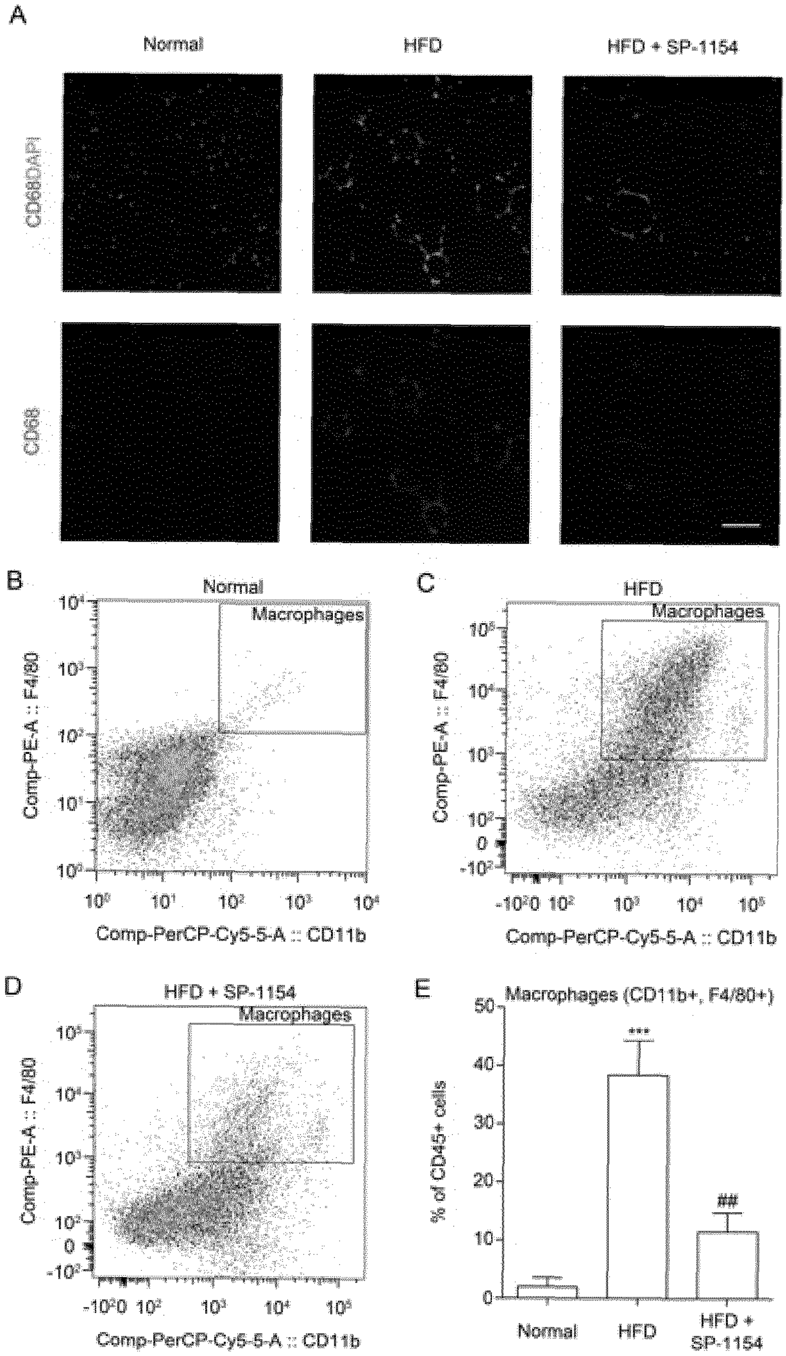
A
Normal
HFD
HFD + SP-1154
CD68DAPI
CD68
B
Normal
Macrophages
Comp-PE-A :: F4/80
Comp-PerCP-Cy5-5-A :: CD11b
C
HFD
Macrophages
Comp-PE-A :: F4/80
Comp-PerCP-Cy5-5-A :: CD11b
D
HFD + SP-1154
Macrophages
Comp-PE-A :: F4/80
Comp-PerCP-Cy5-5-A :: CD11b
E
Macrophages (CD11b+, F4/80+)
% of CD45+ cells
***
##
Normal
HFD
HFD + SP-1154

FIG. 5

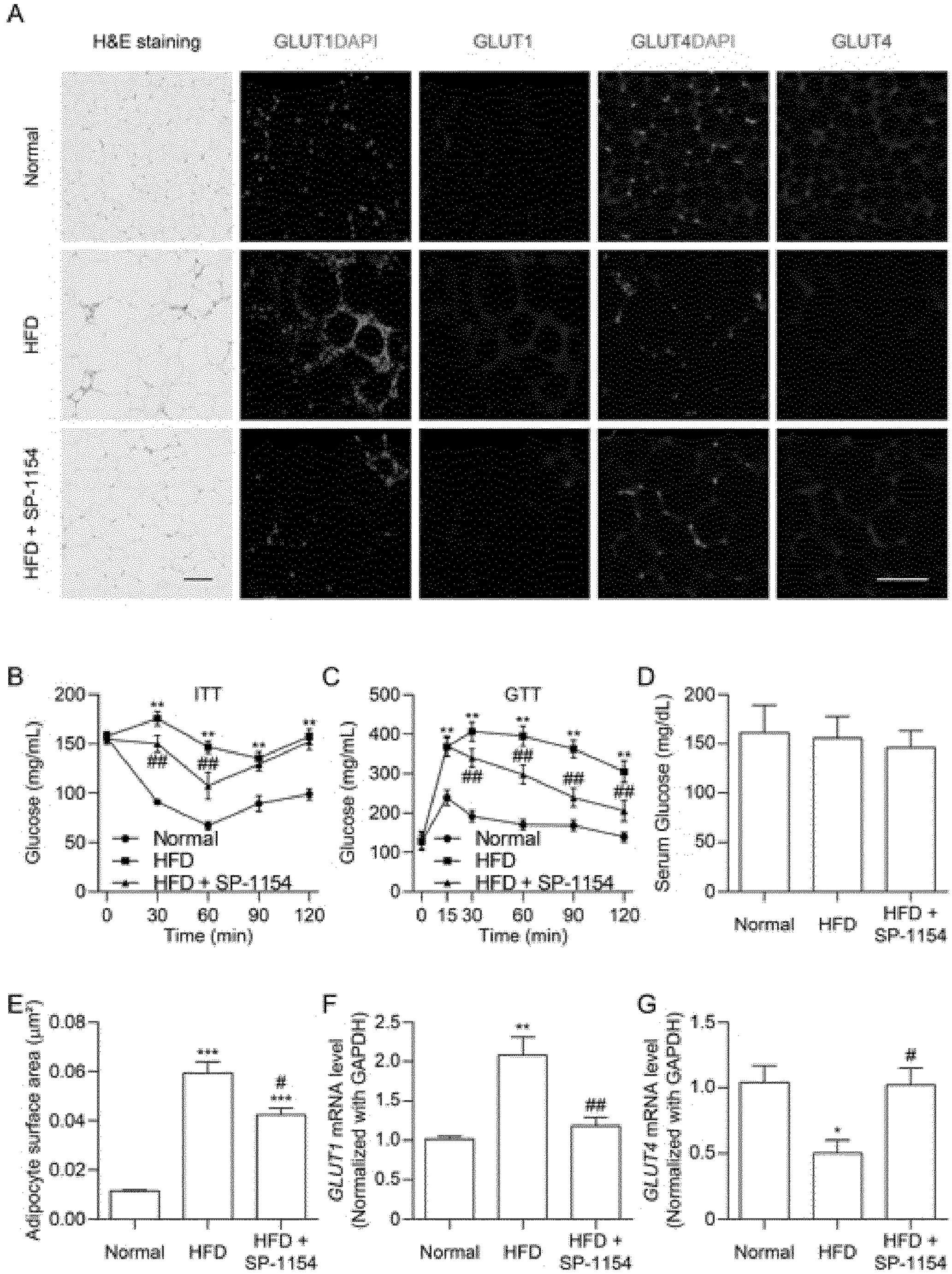
A
H&E staining
GLUT1DAPI
GLUT1
GLUT4DAPI
GLUT4
Normal
HFD
HFD + SP-1154
B
ITT
Glucose (mg/mL)
Time (min)
Normal
HFD
HFD + SP-1154
C
GTT
Glucose (mg/mL)
Time (min)
Normal
HFD
HFD + SP-1154
D
Serum Glucose (mg/dL)
Normal
HFD
HFD + SP-1154
E
Adipocyte surface area (μm²)
Normal
HFD
HFD + SP-1154
F
GLUT1 mRNA level (Normalized with GAPDH)
Normal
HFD
HFD + SP-1154
G
GLUT4 mRNA level (Normalized with GAPDH)
Normal
HFD
HFD + SP-1154

FIG. 6

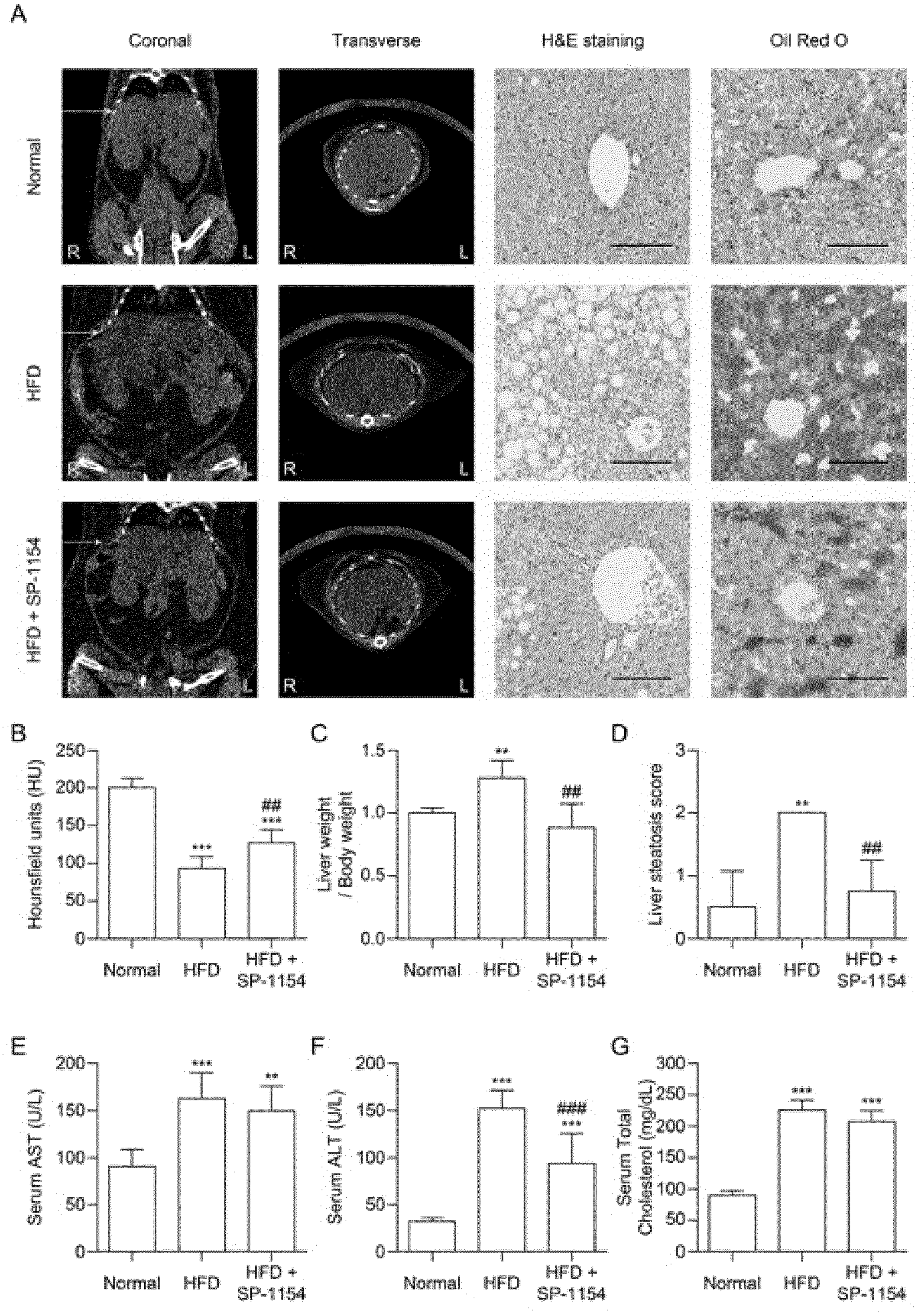
A
Coronal
Transverse
H&E staining
Oil Red O
Normal
HFD
HFD + SP-1154
R
L
B
Hounsfield units (HU)
250
200
150
100
50
0
Normal
HFD
HFD + SP-1154
C
Liver weight / Body weight
1.5
1.0
0.5
0.0
D
Liver steatosis score
3
2
1
0
E
Serum AST (U/L)
F
Serum ALT (U/L)
G
Serum Total Cholesterol (mg/dL)
300
250
200
150
100
50
0
***
**
##
###

**INTERNATIONAL SEARCH REPORT**

International application No. **PCT/KR2022/012956**

**A. CLASSIFICATION OF SUBJECT MATTER**

**A61K 31/122**(2006.01)i; **A61P 3/04**(2006.01)i; **A61P 1/16**(2006.01)i

According to International Patent Classification (IPC) or to both national classification and IPC

**B. FIELDS SEARCHED**

Minimum documentation searched (classification system followed by classification symbols)

A61K 31/122(2006.01); A61K 31/12(2006.01); A61K 31/13(2006.01); A61P 35/00(2006.01); C07C 49/603(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus) & keywords: 베르베논(verbenone), 비만(obesity), 간질환(liver disease), 비알코올성 지방간 질환(non-alcoholic fatty liver disease)

**C. DOCUMENTS CONSIDERED TO BE RELEVANT**

| Category* | Citation of document, with indication, where appropriate, of the relevant passages | Relevant to claim No. |
|---|---|---|
| Y | PAHK, K. et al. A novel CD147 inhibitor, SP-8356, reduces neointimal hyperplasia and arterial stiffness in a rat model of partial carotid artery ligation. Journal of translational medicine. 2019, vol. 17, pp. 1-13. See abstract; page 11, right column; and figure 1. | 1-17 |
| Y | LOU, J. et al. Hepatic CD147 knockout modulates liver steatosis and up-regulates autophagy in high-fat-diet-induced NAFLD mice. Biochemical and biophysical research communications. 2020, vol. 524, pp. 1010-1017. See abstract; and page 1016. | 1-17 |
| A | KR 10-2014-0031568 A (KOREA RESEARCH INSTITUTE OF BIOSCIENCE AND BIOTECHNOLOGY et al.) 13 March 2014 (2014-03-13) See claims 1-11. | 1-17 |
| A | WO 2016-122288 A2 (KOREA UNIVERSITY RESEARCH AND BUSINESS FOUNDATION) 04 August 2016 (2016-08-04) See entire document. | 1-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 November 2022** | **30 November 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2022/012956

C. DOCUMENTS CONSIDERED TO BE RELEVANT

| Category* | Citation of document, with indication, where appropriate, of the relevant passages | Relevant to claim No. |
|---|---|---|
| A | WO 2020-055129 A1 (KOREA UNIVERSITY RESEARCH AND BUSINESS FOUNDATION) 19 March 2020 (2020-03-19)<br>See entire document. | 1-17 |
| A | PAHK, K. et al. SP-1154, a novel synthetic TGF-β inhibitor, alleviates obesity and hepatic steatosis in high-fat diet-induced mice. Biomedicine & pharmacotherapy. 2022, [electronic publication] 20 November 2021, vol. 145, document no. 112441, inner pp. 1-12.<br>See abstract; page 10; and figure 1. | 1-17 |

Form PCT/ISA/210 (second sheet) (July 2022)

**INTERNATIONAL SEARCH REPORT**
**Information on patent family members**

International application No.
**PCT/KR2022/012956**

| Patent document cited in search report | Publication date (day/month/year) | Patent family member(s) | Publication date (day/month/year) |
| --- | --- | --- | --- |
| KR 10-2014-0031568 A | 13 March 2014 | KR 10-1715152 B1 | 13 March 2017 |
| WO 2016-122288 A2 | 04 August 2016 | KR 10-1721068 B1 | 29 March 2017 |
| | | KR 10-2016-0094344 A | 09 August 2016 |
| | | KR 10-2016-0094345 A | 09 August 2016 |
| | | WO 2016-122288 A3 | 10 November 2016 |
| | | WO 2016-122288 A9 | 29 December 2016 |
| | | WO 2016-122289 A2 | 04 August 2016 |
| | | WO 2016-122289 A3 | 27 October 2016 |
| WO 2020-055129 A1 | 19 March 2020 | KR 10-2020-0029772 A | 19 March 2020 |
| | | KR 10-2145416 B1 | 19 August 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20190026


### Non-patent literature cited in the description


- **BYRNE CD ; TARGHER G.** NAFLD: a multisystem disease. *Journal of hepatology.,* 2015, vol. 62, S47-S64
- **YOUNOSSI ZM ; BLISSETT D ; BLISSETT R ; HENRY L ; STEPANOVA M ; YOUNOSSI Y et al.** The economic and clinical burden of nonalcoholic fatty liver disease in the United States and Europe. *Hepatology.,* 2016, vol. 64, 1577-86
- **YOUNOSSI ZM ; KOENIG AB ; ABDELATIF D ; FAZEL Y ; HENRY L ; WYMER M.** Global epidemiology of nonalcoholic fatty liver disease-meta-analytic assessment of prevalence, incidence, and outcomes. *Hepatology.,* 2016, vol. 64, 73-84
- **KHAN RS ; BRIL F ; CUSI K ; NEWSOME PN.** Modulation of insulin resistance in nonalcoholic fatty liver disease. *Hepatology.,* 2019, vol. 70, 711-24
- **FRIEDMAN SL ; NEUSCHWANDER-TETRI BA ; RINELLA M ; SANYAL AJ.** Mechanisms of NAFLD development and therapeutic strategies. *Nature medicine.,* 2018, vol. 24, 908-22
- **WANG Y ; TANG B ; LONG L ; LUO P ; XIANG W ; LI X et al.** Improvement of obesity-associated disorders by a small-molecule drug targeting mitochondria of adipose tissue macrophages. *Nature communications.,* 2021, vol. 12, 1-16
- **LEE M-J.** Transforming growth factor beta superfamily regulation of adipose tissue biology in obesity. *Biochimica et Biophysica Acta (BBA)-Molecular Basis of Disease.,* 2018, vol. 1864, 1160-71
- **SOUSA-PINTO B ; GONCALVES L ; RODRIGUES AR ; TADA I ; ALMEIDA H ; NEVES D et al.** Characterization of TGF-β expression and signaling profile in the adipose tissue of rats fed with high-fat and energy-restricted diets. *The Journal of nutritional biochemistry,* 2016, vol. 38, 107-15
- **TAN C ; CHONG H ; TAN E ; TAN N.** Getting 'Smad' about obesity and diabetes. *Nutrition & diabetes.,* 2012, vol. 2, 29
- **TAN CK ; LEUENBERGER N ; TAN MJ ; YAN YW ; CHEN Y ; KAMBADUR R et al.** Smad3 deficiency in mice protects against insulin resistance and obesity induced by a high-fat diet. *Diabetes.,* 2011, vol. 60, 464-76
- **YADAV H ; QUIJANO C ; KAMARAJU AK ; GAVRILOVA O ; MALEK R ; CHEN W et al.** Protection from obesity and diabetes by blockade of TGF-β/Smad3 signaling. *Cell metabolism.,* 2011, vol. 14, 67-79
- **JU C ; SONG S ; HWANG S ; KIM C ; KIM M ; GU J et al.** Discovery of novel (1S)-(-)-verbenone derivatives with anti-oxidant and anti-ischemic effects. *Bioorganic & medicinal chemistry letters.,* 2013, vol. 23, 5421-5
- **NGUYEN HT ; REYES-ALCARAZ A ; YONG HJ ; NGUYEN LP ; PARK H-K ; INOUE A et al.** CXCR7: a β-arrestin-biased receptor that potentiates cell migration and recruits β-arrestin2 exclusively through Gβγ subunits and GRK2. *Cell & bioscience.,* 2020, vol. 10, 1-19
- **BACHMANOV AA ; REED DR ; BEAUCHAMP GK ; TORDOFF MG.** Food intake, water intake, and drinking spout side preference of 28 mouse strains. *Behavior genetics.,* 2002, vol. 32, 435-43
- **PAHK K ; KIM EJ ; JOUNG C ; SEO HS ; KIM S.** Exercise training reduces inflammatory metabolic activity of visceral fat assessed by 18F-FDG PET/CT in obese women. *Clinical Endocrinology.,* 2020
- **PAHK K ; KIM EJ ; JOUNG C ; SEO HS ; KIM S.** Association of glucose uptake of visceral fat and acute myocardial infarction: a pilot 18 F-FDG PET/CT study. *Cardiovascular Diabetology.,* 2020, vol. 19, 1-11
- **JIMENEZ-GOMEZ Y ; MATTISON JA ; PEARSON KJ ; MARTIN-MONTALVO A ; PALACIOS HH ; SOSSONG AM et al.** Resveratrol improves adipose insulin signaling and reduces the inflammatory response in adipose tissue of rhesus monkeys on high-fat, high-sugar diet. *Cell metabolism.,* 2013, vol. 18, 533-45
- **LEE ES ; KWON M-H ; KIM HM ; WOO HB ; AHN CM ; CHUNG CH.** Curcumin analog CUR5-8 ameliorates nonalcoholic fatty liver disease in mice with high-fat diet-induced obesity. *Metabolism.,* 2020, vol. 103, 154015

- **LIVAK KJ ; SCHMITTGEN TD.** *Analysis of relative gene expression data using real-time quantitative PCR and the 2- ΔΔCT method, methods,* 2001, vol. 25, 402-8
- **ELSEGOOD CL ; CHANG M ; JESSUP W ; SCHOLZ GM ; HAMILTON JA.** Glucose Metabolism Is Required for Oxidized LDL-Induced Macrophage Survival: Role of PI3K and Bcl-2 Family Proteins. *Arteriosclerosis, thrombosis, and vascular biology.,* 2009, vol. 29, 1283-9
- **MORAES-VIEIRA PM ; SAGHATELIAN A ; KAHN BB.** GLUT4 expression in adipocytes regulates de novo lipogenesis and levels of a novel class of lipids with antidiabetic and anti-inflammatory effects. *Diabetes.,* 2016, vol. 65, 1808-15
- **VIGLINO D ; MARTIN M ; ALMERAS N ; DESPRÉS J-P ; COXSON HO ; PÉPIN J-L et al.** Low Liver Density Is Linked to Cardiovascular Comorbidity in COPD: An ECLIPSE Cohort Analysis. *International Journal of Chronic Obstructive Pulmonary Disease.,* 2019, vol. 14, 3053
- **UM MY ; MOON MK ; AHN J ; HA TY.** Coumarin attenuates hepatic steatosis by down-regulating lipogenic gene expression in mice fed a high-fat diet. *British journal of nutrition.,* 2013, vol. 109, 1590-7
- **FENG D ; ZOU J, SU D ; MAI H ; ZHANG S ; LI P et al.** Curcumin prevents high-fat diet-induced hepatic steatosis in ApoE-/- mice by improving intestinal barrier function and reducing endotoxin and liver TLR4/NF-κB inflammation. *Nutrition & metabolism.,* 2019, vol. 16, 1-11
- **CILDIR G ; AKINCILAR SC ; TERGAONKAR V.** Chronic adipose tissue inflammation: all immune cells on the stage. *Trends in molecular medicine.,* 2013, vol. 19, 487-500
- **XIE L ; ORTEGA MT ; MORA S ; CHAPES SK.** Interactive changes between macrophages and adipocytes. *Clinical and Vaccine Immunology.,* 2010, vol. 17, 651-9
- **LUMENG CN ; DEYOUNG SM ; SALTIEL AR.** Macrophages block insulin action in adipocytes by altering expression of signaling and glucose transport proteins. *American Journal of Physiology-Endocrinology and Metabolism,* 2007, vol. 292, E166-E74
- **CHALASANI N ; YOUNOSSI Z ; LAVINE JE ; CHARLTON M ; CUSI K ; RINELLA M et al.** The diagnosis and management of nonalcoholic fatty liver disease: practice guidance from the American Association for the Study of Liver Diseases. *Hepatology.,* 2018, vol. 67, 328-57
- **ADAMS LA ; LYMP JF ; SAUVER JS ; SANDERSON SO ; LINDOR KD ; FELDSTEIN A et al.** The natural history of nonalcoholic fatty liver disease: a population-based cohort study. *Gastroenterology,* 2005, vol. 129, 113-21
- **MUSOLINO V ; GLIOZZI M ; SCARANO F ; BOSCO F ; SCICCHITANO M ; NUCERA S et al.** Bergamot polyphenols improve dyslipidemia and pathophysiological features in a mouse model of non-alcoholic fatty liver disease. *Scientific reports.,* 2020, vol. 10, 1-14
- **NAM J-S ; TERABE M ; MAMURA M ; KANG M-J ; CHAE H ; STUELTEN C et al.** An anti-transforming growth factor β antibody suppresses metastasis via cooperative effects on multiple cell compartments. *Cancer research,* 2008, vol. 68, 3835-43
- **TAKAHASHI K ; AKATSU Y ; PODYMA-INOUE KA ; MATSUMOTO T ; TAKAHASHI H ; YOSHIMATSU Y et al.** Targeting all transforming growth factor-β isoforms with an Fc chimeric receptor impairs tumor growth and angiogenesis of oral squamous cell cancer. *Journal of Biological Chemistry,* 2020, vol. 295, 12559-72
- **CHAMES P ; VAN REGENMORTEL M ; WEISS E ; BATY D.** Therapeutic antibodies: successes, limitations and hopes for the future. *British journal of pharmacology.,* 2009, vol. 157, 220-33
- **BELL MR ; ENGLEKA MJ ; MALIK A ; STRICKLER JE.** To fuse or not to fuse: what is your purpose. *Protein Science.,* 2013, vol. 22, 1466-77
- **SANTIAGO B ; GUTIERREZ-CAÑAS I ; DOTOR J ; PALAO G ; LASARTE JJ ; RUIZ J et al.** Topical application of a peptide inhibitor of transforming growth factor-β1 ameliorates bleomycin-induced skin fibrosis. *Journal of Investigative Dermatology.,* 2005, vol. 125, 450-5
- **GALLO-OLLER G ; VOLLMANN-ZWERENZ A ; MELÉNDEZ B ; REY JA ; HAU P ; DOTOR J et al.** P144, a Transforming Growth Factor beta inhibitor peptide, generates antitumoral effects and modifies SMAD7 and SKI levels in human glioblastoma cell lines. *Cancer letters,* 2016, vol. 381, 67-75
- **HUANG C-Y ; CHUNG C-L ; HU T-H ; CHEN J-J ; LIU P-F ; CHEN C-L.** Recent progress in TGF-β inhibitors for cancer therapy. *Biomedicine & Pharmacotherapy.,* 2021, vol. 134, 111046